(19) Europäisches Patentamt
European Patent Office
Office européen des brevets

(11) EP 1 345 902 B1

(12) # EUROPÄISCHE PATENTSCHRIFT

(45) Veröffentlichungstag und Bekanntmachung des Hinweises auf die Patenterteilung:
**01.06.2005 Patentblatt 2005/22**

(21) Anmeldenummer: **01273771.4**

(22) Anmeldetag: **07.12.2001**

(51) Int Cl.[7]: **C07D 213/38**, C07D 307/52, C07D 333/20, A61P 9/00, A61P 1/00, A61K 31/381, A61K 31/417, A61K 31/40, C07D 277/28, A61K 31/426, C07D 241/12, A61K 31/4965, C07D 239/26, A61K 31/505, C07D 237/08, A61K 31/50, C07D 231/12, C07D 231/10, A61K 31/415, C07D 333/28, C07D 333/44

(86) Internationale Anmeldenummer:
**PCT/EP2001/014422**

(87) Internationale Veröffentlichungsnummer:
**WO 2002/066431 (29.08.2002 Gazette 2002/35)**

(54) **SUBSTITUIERTE HETEROCYCLO-NORBORNYLAMINO-DERIVATE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**

SUBSTITUTED HETEROCYCLO-NORBORNYLAMINE DERIVATIVES, METHOD FOR PRODUCING THE SAME, THEIR USE AS MEDICAMENT OR DIAGNOSTIC AGENT AND MEDICAMENT CONTAINING THE SAME

DERIVES HETEROCYCLO-NORBONYLAMINO SUBSTITUES, LEUR PROCEDE DE PRODUCTION, LEUR UTILISATION COMME MEDICAMENT OU AGENT DE DIAGNOSTIC ET MEDICAMENT LES CONTENANT

(84) Benannte Vertragsstaaten:
**AT BE CH CY DE DK ES FI FR GB GR IE IT LI LU MC NL PT SE TR**

(30) Priorität: **19.12.2000 DE 10063294**

(43) Veröffentlichungstag der Anmeldung:
**24.09.2003 Patentblatt 2003/39**

(73) Patentinhaber: **Aventis Pharma Deutschland GmbH**
**65929 Frankfurt am Main (DE)**

(72) Erfinder:
- **HEINELT, Uwe**
  **65187 Wiesbaden (DE)**
- **LANG, Hans-Jochen**
  **65719 Hofheim (DE)**
- **WIRTH, Klaus**
  **65830 Kriftel (DE)**
- **JANSEN, Hans-Willi**
  **65527 Niedernhausen (DE)**

(56) Entgegenhaltungen:
**WO-A-01/44164**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 345 902 B1

## Beschreibung

Die Erfindung betrifft substituierte Heterocyclo-Norbomylamino-Derivate mit exo-konfiguriertem Stickstoff und endo-anelliertem Fünf- oder Sechsring der Formel I oder mit exo-konfiguriertem Stickstoff und exo-anelliertem Fünf- oder Sechsring der Formel I a,

I

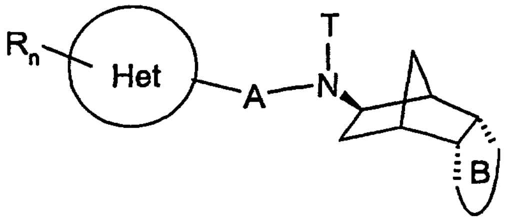

I a

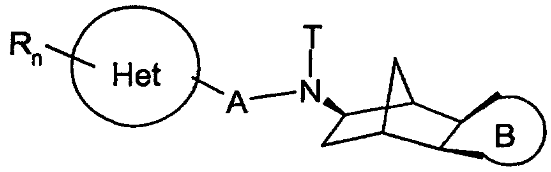

worin bedeuten:

- A $(C_1\text{-}C_4)$- Alkylen;
- T $(C_1\text{-}C_4)$- Alkyl oder H;
- B einen gesättigten oder ungesättigten Kohlenstoff-Fünf- oder Sechsring, der unsubstituiert ist oder substituiert ist mit 1 - 3 Substituenten ausgewählt aus der Gruppe bestehend aus Oxo, Hydroxy, $(C_1\text{-}C_4)$- Alkoxy und $(C_1\text{-}C_4)$- Alkyl;
- Het einen 5- oder 6-gliedrigen, gesättigten oder ungesättigten Heterocyclus, der bis zu vier gleiche oder verschiedene Heteroatome ausgewählt aus der Gruppe bestehend aus O, S, N und Se enthält;
- R OH, F, Cl, Br, I, CN, $NO_2$, Phenyl, $CO_2R1$, $(C_1\text{-}C_4)$- Alkyl, $(C_1\text{-}C_4)$- Alkoxy, Amino, $(C_1\text{-}C_4)$- Alkylamino, Di-$(C_1\text{-}C_4)$- alkylamino, Amino-$(C_1\text{-}C_4)$- alkyl,
  wobei die Alkylreste unsubstituiert sind oder ganz oder teilweise durch Fluor substituiert sind;

  R1 H oder $(C_1\text{-}C_4)$-Alkyl, das unsubstituiert oder ganz oder teilweise durch Fluor substituiert ist;

- n 0, 1, 2, 3 oder 4,
  wobei, wenn n = 2, 3 oder 4 ist, die Substituenten R unabhängig voneinander sind;

sowie deren pharmazeutisch verträgliche Salze oder Trifluoracetate.

Bevorzugt sind Verbindungen mit exo-konfiguriertem Stickstoff und endo-anelliertem Kohlenstoff-Fünf- oder Sechsring der Formel I oder mit exo-konfiguriertem Stickstoff und exo-anelliertem Kohlenstoff Fünf- oder Sechsring der Formel I a, worin bedeuten:

- A $(C_1\text{-}C_2)$- Alkylen;
- T H oder Methyl;
- B einen gesättigten oder ungesättigten Kohlenstoff-Fünf- oder Sechsring;
- Het einen 5- oder 6-gliedrigen, gesättigten oder ungesättigten Heterocyclus, der bis zu drei gleiche oder verschiedene Heteroatome ausgewählt aus der Gruppe bestehend aus O, S und N enthält;
- R F, Cl, Br, Iod, Amino, Hydroxymethyl, OH, Phenyl, $CO_2R1$, $(C_1\text{-}C_4)$- Alkyl oder $(C_1\text{-}C_4)$- Alkoxy,
  wobei die Alkylreste unsubstituiert oder ganz oder teilweise durch Fluor substituiert sind;

  R1 H oder $(C_1\text{-}C_4)$-Alkyl, wobei der Alkylrest unsubstituiert oder ganz oder teilweise durch Fluor substituiert ist;

- n 0, 1, 2 oder 3,
  wobei, wenn n = 2 oder 3 ist, die entsprechenden Substituenten R unabhängig voneinander sind;

sowie deren pharmazeutisch verträgliche Salze oder Trifluoracetate.

Besonders bevorzugt sind Verbindungen mit exo-konfiguriertem Stickstoff und endo-anelliertem Kohlenstoff-Fünf- oder Sechsring der Formel I oder mit exo-konfiguriertem Stickstoff und exo-anelliertem Kohlenstoff-Fünf- oder Sechsring der Formel I a, worin bedeuten:

A $(C_1-C_2)$- Alkylen;
T Wasserstoff;
B einen gesättigten oder ungesättigten Kohlenstoff-Fünf- oder Sechsring;
Het einen 5- oder 6-gliedrigen, gesättigten oder ungesättigten Heterocyclus, der bis zu zwei gleiche oder verschiedene Heteroatome ausgewählt aus der Gruppe bestehend aus O, S und N enthält ;
R F, Cl, Br, $(C_1-C_4)$- Alkoxy oder $(C_1-C_4)$- Alkyl,
wobei die Alkylreste unsubstituiert sind oder ganz oder teilweise durch Fluor substituiert;
n 0, 1 oder 2, wobei, wenn n = 2, die entsprechenden Substituenten R unabhängig voneinander sind;

sowie deren pharmazeutisch verträgliche Salze oder Trifluoracetate.

Ganz besonders bevorzugt sind die folgenden Verbindungen mit exo-konfiguriertem Stickstoff und endo-anelliertem Kohlenstoff-Fünf- oder Sechsring der Formel I oder mit exo-konfiguriertem Stickstoff und exo-anelliertem Kohlenstoff-Fünfring der Formel I a :

exo/exo-(Octahydro-4,7-methano-inden-5-yl)-pyridin-3-ylmethyl-amin,
(rac)-exo/endo-(Octahydro-4,7-methano-inden-5-yl)-pyridin-3-ylmethyl-amin,
(+)-exo/endo-(Octahydro-4,7-methano-inden-5-yl)-pyridin-3-ylmethyl-amin,
(-)-exo/endo-(Octahydro-4,7-methano-inden-5-yl)-pyridin-3-ylmethyl-amin,
(rac)-exo/endo-(Octahydro-4,7-methano-inden-5-yl)-pyrazin-2-ylmethyl-amin,
(+)-exo/endo-(Octahydro-4,7-methano-inden-5-yl)-pyrazin-2-ylmethyl-amin,
(-)-exo/endo-(Octahydro-4,7-methano-inden-5-yl)-pyrazin-2-ylmethyl-amin,
exo/endo-(Decahydro-1,4-methano-naphthalen-2-yl)-pyrazin-2-ylmethyl-amin,
exo/endo-(Octahydro-4,7-methano-inden-5-yl)-thiophen-2-ylmethyl-amin,
exo/endo-(Octahydro-4,7-methano-inden-5-yl)-thiophen-3-ylmethyl-amin,
exo/endo-(3a,4,5,6,7,7a-Hexahydro-3H-4,7-methano-inden-5-yl)-pyridin-3-yl-methyl-amin,
exo/endo-(3a,4,5,6,7,7a-Hexahydro-1H-4,7-methano-inden-5-yl)-pyridin-3-yl-methylamin,
exo/endo-Furan-3-ylmethyl-(octahydro-4,7-methano-inden-5-yl)-amin,
exo/endo-Furan-2-ylmethyl-(octahydro-4,7-methano-inden-5-yl)-amin,
exo/endo-(Decahydro-1,4-methano-naphthalen-2-yl)-pyridin-3-ylmethyl-amin,
exo/endo-(Octahydro-4,7-methano-inden-5-yl)-(1H-pyrrol-2-ylmethyl)-amin,
exo/endo-(Octahydro-4,7-methano-inden-5-yl)-pyrimidin-5-ylmethyl-amin

sowie deren pharmazeutisch verträgliche Salze oder Trifluoracetate.

Außerordentlich besonders bevorzugt sind die folgenden Verbindungen mit exo-konfiguriertem Stickstoff und endo-anelliertem Kohlenstoff-Fünf- oder Sechsring der Formel I oder mit exo-konfiguriertem Stickstoff und exo-anelliertem Kohlenstoff-Fünfring der Formel I a :

exo/exo-(Octahydro-4,7-methano-inden-5-yl)-pyridin-3-ylmethyl-amin,
(rac)-exo/endo-(Octahydro-4,7-methano-inden-5-yl)-pyridin-3-ylmethyl-amin,
(+)-exo/endo-(Octahydro-4,7-methano-inden-5-yl)-pyridin-3-ylmethyl-amin,
(-)-exo/endo-(Octahydro-4,7-methano-inden-5-yl)-pyridin-3-ylmethyl-amin,
(rac)-exo/endo-(Octahydro-4,7-methano-inden-5-yl)-pyrazin-2-ylmethyl-amin,
(+)-exolendo-(Octahydro-4,7-methano-inden-5-yl)-pyrazin-2-ylmethyl-amin,
exo/endo-(Octahydro-4,7-methano-inden-5-yl)-thiophen-2-ylmethyl-amin,
exo/endo-(3a,4,5,6,7,7a-Hexahydro-3H-4,7-methano-inden-5-yl)-pyridin-3-yl-methyl-amin,
exo/endo-(3a,4,5,6,7,7a-Hexahydro-1H-4,7-methano-inden-5-yl)-pyridin-3-yl-methylamin,
exo/endo-(Decahydro-1,4-methano-naphthalen-2-yl)-pyridin-3-ylmethyl-amin,
exo/endo-(Octahydro-4,7-methano-inden-5-yl)-(1H-pyrrol-2-ylmethyl)-amin,
exolendo-(Octahydro-4,7-methano-inden-5-yl)-pyrimidin-5-ylmethyl-amin

sowie deren pharmazeutisch verträgliche Salze oder Trifluoracetate.

Als Säureadditionssalze kommen dabei Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate. Diese Gruppe entspricht auch den physiologisch akzeptablen Anionen; aber auch Trifuoracetate.

Enthält die Verbindung der Formel I oder I a ein oder mehrere Asymmetriezentren, so können diese sowohl S- als auch R-konfiguriert sein. Die Verbindungen können als optische Isomere, Diastereomere, Racemate oder Gemische derselben vorliegen. Allerdings muss der Aminosubstituent am Norbornylsystem exo-ständig und der Ring endo- beziehungsweise exo-anelliert sein.

Die genannten Alkyl- oder Alkylen- Reste können sowohl geradkettig als auch verzweigt vorliegen.

Als Heterocyclen kommen unter anderem in Frage:

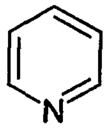 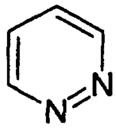 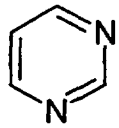 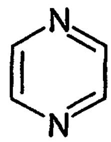 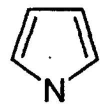 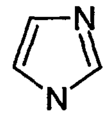 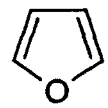 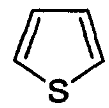

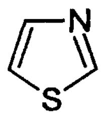 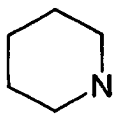 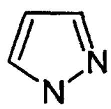 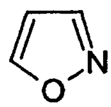 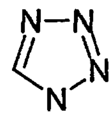 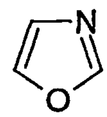 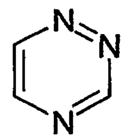

Die Erfindung betrifft weiterhin ein Verfahren zur Herstellung der Verbindungen der Formel I oder I a, dadurch gekennzeichnet, dass man

a) eine Verbindung der Formel II oder II a

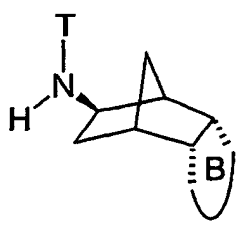 II

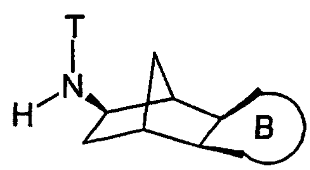

II a

mit einer Verbindung der Formel III

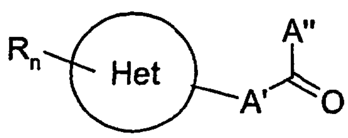 III

in Gegenwart geeigneter Reduktionsmittel und möglicherweise auch Lewis-Säuren direkt zu Verbindungen der Formel I oder I a umsetzt, worin T, B, Het und $R_n$ die oben angegebene Bedeutung besitzen, während unabhängig voneinander A' einer Bindung oder ($C_1$-$C_3$)- Alkyl und A" H oder ($C_1$-$C_3$)-Alkyl entspricht und A' und A" zusammen mit dem Kohlenstoffatom der Carbonylgruppe so viele Kohlenstoffatome repräsentieren wie das oben beschriebene A;

oder

b) das aus Verbindungen der Formeln II oder II a und III gebildete Intermediat der Formel IV oder IV a,

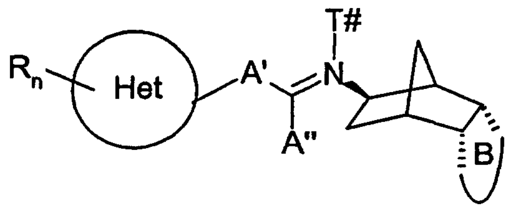
IV

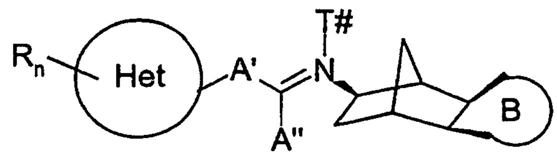
IV a

worin T# einem freien Elektronenpaar oder ($C_1$-$C_4$)-Alkyl entspricht und bei letzterer Bedeutung ein Onium-Stickstoff gebildet wird, dem ein Gegenion wie beispielsweise Chlorid oder Tosylat zugeordnet ist,
isoliert und dann mit geeigneten Reduktionsmitteln in die Verbindungen der Formel I oder I a überführt,
oder
c) eine Verbindung der Formel II oder II a mit einem Alkylierungsmittel der Formel V,

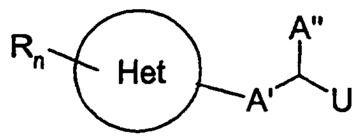
V

in der U für eine nukleophil substituierbare Gruppe steht - wie Cl, Br, J, Mesylat oder Tosylat - und die anderen Reste wie oben beschrieben definiert sind, aber hier dem Kohlenstoffatom der Carbonylgruppe das Kohlenstoffatom, an das U gebunden ist, entspricht,
umsetzt
oder
d) Carbonsäureamide der Formel VI oder VI a,

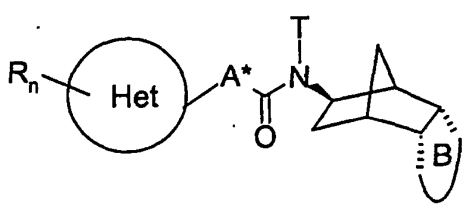
VI

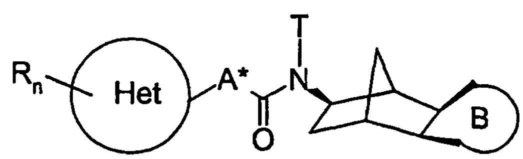
VI a

worin A* einer Bindung oder ($C_1$-$C_3$)-Alkyl entspricht und die anderen Reste, wie oben beschrieben, definiert sind, ,
zu den entsprechenden Aminen reduziert;
oder
e) Verbindungen der Formel I oder I a, in denen T Wasserstoff entspricht, mit Alkylierungsmitteln der Formel VII,

T*-U VII

worin T* ($C_1$-$C_4$)- Alkyl bedeutet und U oben beschriebene Bedeutung besitzt, alkyliert, so dass aus dieser Umsetzung tertiäre Amine hervorgehen;
oder
f) einen Dicyclopentadienylplatin-Komplex der Formel VIII

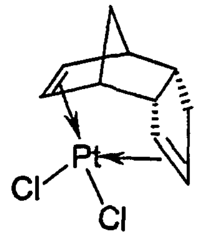

VIII

mit Aminen vom Typ der Formel IX,

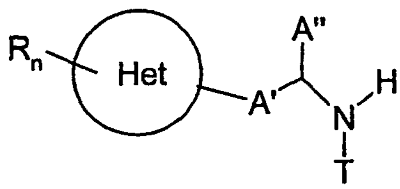

IX

worin T, $R_n$ und Het die oben angegebene Bedeutung besitzen, während unabhängig voneinander A' einer Bindung oder ($C_1$-$C_3$)-Alkyl und A" H oder ($C_1$-$C_3$)-Alkyl entspricht und A' und A" zusammen mit dem Kohlenstoffatom, an welches das Stickstoffatom gebunden ist, so viele Kohlenstoffatome repräsentieren wie das oben beschriebene A,

umsetzt und anschließend das gebildete Zwischenprodukt zu Verbindungen der Formel I reduziert (J. K. Stille, D. B. Fox JACS 1970 (92), 1274),
die man gegebenenfalls in das pharmazeutisch verträgliche Salz oder Trifluoracetat überführt.
Es ist schon vorgeschlagen worden, dass Phenylalkyl-substituierte Norbomylamino-Derivate effektive Inhibitoren des Natrium-Protonen Austauschers, Subtyp 3 (NHE3) sind. Dabei zeigte sich, dass von mehreren Stereoisomeren die Verbindungen mit exo/endo-konfiguriertem Octahydro-4,7-methano-inden-5ylamin-Baustein, in dem der Stickstoff exo-ständig und der Fünfring endo-anelliert ist, sich als besonders aktive NHE3-Inhibitoren erwiesen. Substanzen mit exolexo-konfiguriertem Octahydro-4,7-methano-inden-5ylamin-Baustein zeigten ebenfalls noch deutlich NHE3-inhibierende Wirkung, während die entsprechenden endo/endo- und endo/exo-Derivate am NHE3 deutlich wirkschwächer waren (Deutsche Offenlegungsschrift 199 60 204 A 1 - HMR 99/L 073).

Überraschend wurde nun gefunden, dass der aromatische Teil des Phenylalkyl-Substituenten durch heteroaromatische Ringe unter Erhalt der NHE3-inhibierenden Wirkung substituiert werden kann.

Gegenüber den seit längerem bekannten Inhibitoren des Natrium-Protonen-Austauschers, Subtyp 3 nach EP-OS 825 178 (HOE 96/F226), die retativ polare Strukturen repräsentieren und dem Acylguanidintyp entsprechen (J.-R. Schwark et al.

Eur. J. Physiol (1998) 436:797), handelt es sich bei den erfindungsgemäßen Verbindungen wie bei den vorgeschlagenen Verbindungen vom Phenylalkylnorbomylamin-Typ (DE 199 60 204.2 - HMR 99 / L 073) um überraschend lipophile Substanzen, die nicht vom Acylguanidintyp sind. Es handelt sich unseren Recherchen zufolge nach den soeben genannten Acylguanidinen, dem Squalamin (M. Donowitz et al. Am. J. Physiol. 276 (Cell Physiol. 45): C136 - C144), das allerdings nicht unmittelbar, sondern erst nach etwa einer Stunde seine maximale Wirkstärke erreicht, und den obigen Phenylalkyl-norbornylamino-Derivaten erst um die vierte Stoffklasse von NHE3-Inhibitoren, die bislang bekannt wurde. Gegenüber den obigen Acylguanidinen zeichnen sie sich durch überlegene Membrangängigkeit aus. Gegenüber dem Squalamin durch rascheren Wirkeintritt.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Die erfindungsgemäßen Verbindungen der Formel I oder I a eignen sich zur Verwendung als Antihypertensiva für die Behandlung der primären und sekundären Hypertonie.
Außerdem können die Verbindungen alleine oder in Verbindung mit NHE-Inhibitoren anderer Subtypspezifität akut oder chronisch sauerstoffmangelversorgte Organe durch Verringerung oder Verhinderung ischämisch induzierter Schäden schützen. Sie eignen sich somit als Arzneimittel z.B. für operative Eingriffe (z.B. bei Organ-Transplantationen

von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder akutes und chronisches Nierenversagen. Besonders vorteilhaft lassen sich ischämisch induzierte Schäden am Darm vermeiden.

Entsprechend ihrer protektiven Wirkung gegen ischämisch induzierte Schäden sind die Verbindungen potenziell auch als Arzneimittel zur Behandlung von Ischämien des Nervensystems, insbesondere des ZNS, geeignet, wobei sie z. B. zur Behandlung des Schlaganfalls oder des Himödems geeignet sind. Darüber hinaus eignen sich die erfindungsgemäßen Verbindungen der Formel I oder I a ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Zusätzlich erhöhen die Verbindungen den Muskeltonus der oberen Atemwege, so dass das Schnarchen unterdrückt wird.

Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Es hat sich gezeigt, dass die erfindungsgemäßen Verbindungen eine milde abführende Wirkung besitzen und demzufolge vorteilhaft als Abführmittel oder bei drohender Darmverstopfung verwendet werden können, wobei die Verhinderung der mit Verstopfungen im Darmbereich einhergehenden ischämischen Schäden besonders vorteilhaft ist.

Weiterhin ermöglicht der Einsatz der erfindungsgemäßen Verbindungen der Formel I oder I a der Gallenstein-Bildung vorzubeugen.

Zusätzlich zeigen die erfindungsgemäßen Verbindungen der Formel I oder I a eine Wirkung gegen Ektoparasiten.

Darüber hinaus können die erfindungsgemäßen Verbindungen der Formel I oder I a eine inhibierende Wirkung auf die Proliferationen von Zellen, beispielsweise der Fibroblasten-Zellproliferation und der Proliferation der glatten Gefäßmuskelzellen ausüben. Deshalb kommen die Verbindungen der Formel I oder I a als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, endotheliale Dysfunktion, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters der bei zahlreichen Erkrankungen (essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw. Darüber hinaus sind die Verbindungen der Formel I oder I a für die präventive Therapie zur Verhinderung der Genese des Bluthochdrucks, beispielweise der essentiellen Hypertonie, geeignet.

Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Die erfindungsgemäßen Verbindungen können daher zur Prophylaxe und zur Regression von atherosklerotischen Veränderungen herangezogen werden, indem sie einen kausalen Risikofaktor ausschalten. Mit diesem Schutz der Gefäße gegen das Syndrom der endothelialen Dysfunktion sind Verbindungen der Formel I oder I a wertvolle Arzneimittel zur Prävention und zur Behandlung koronarer Gefäßspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens, zur Herstellung eines Medikaments zur Blutdrucksenkung, zur Herstellung eines Medikaments mit abführender Wirkung zur Prävention und Behandlung intestinaler Verstopfungen; zur Herstellung eines Medikaments zur Prävention und Behandlung von Erkrankungen, die durch Ischämie und Reperfusion von zentralen und peripheren Organen ausgelöst werden, wie das akute Nierenversagen, der Schlaganfall, endogene Schockzustände, Darmerkrankungen etc.; zur Herstellung eines Medikaments zur Behandlung von Hypercholesterinämie; zur Herstellung eines Medikaments zur Prävention der Atherogenese und der Atherosklerose; zur Herstellung eines Medikaments zur Prävention und Behandlung von Krankheiten, die durch erhöhte Cholesterinspiegel ausgelöst werden; zur Herstellung eines Medikaments zur Prävention und Behandlung von Krankheiten, die durch endotheliale Dysfunktion ausgelöst werden; zur Herstellung eines Medikaments zur Behandlung des Befalls durch Ektoparasiten; zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I oder I a mit einem blutfettspiegelsenkenden Wirkstoff, bevorzugt mit einem HMG-CoA-Reduktaseinhibitor (z. B. Lovastatin oder Pravastatin), wobei letzterer eine hypolipidämische Wirkung herbeiführt und dadurch die hypolipidämischen Eigenschaften des NHE-Inhibitors der Formel I oder I a steigert, erweist sich als günstige Kombination mit verstärkter Wirkung und vermindertem Wirkstoffeinsatz.

Beansprucht wird die Gabe von Natrium-Protonen-Austausch-Hemmern der Formel I oder I a als neuartige Arzneimittel zur Senkung erhöhter Blutfettspiegel, sowie die Kombination von Natrium-Protonen-Austausch-Hemmem mit blutdrucksenkenden und/oder hypolipidämisch wirkenden Arzneimitteln.

Arzneimittel, die eine Verbindung I oder I a enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I oder I a können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositorien-Grundlagen, Tablettenhilfsstoffen und anderen Wirkstoffträgem können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I oder I a in einem pharmazeutisch unbedenklichen Lösungsmittel, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I oder I a und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I oder I a bei einem etwa 75 kg schweren Patienten mindestens 0,001 mg/kg, vorzugsweise 1 - 10 mg/kg, bis höchstens 100 mg/kg Körpergewicht. Bei akuten Ausbrüchen der Krankheiten können auch noch höhere und vor allem häufigere Dosierungen notwendig sein, z. B. bis zu 4 Einzeldosen pro Tag. Insbesondere bei i. v. Anwendung, etwa bei einem Infarktpatienten auf der Intensivstation können bis zu 200 mg pro Tag notwendig werden.

Experimenteller Teil:

## Verwendete Abkürzungen:

| | |
|---|---|
| CI | chemische Ionisation |
| DIP | Diisopropylether |
| EE | Ethylacetat |
| ES | Elektrospray |
| FAB | Fast-Atom Bombardment |
| h | Stunden |
| HCl | Salzsäure |
| HPLC-RT | HPLC-Retentionszeit |

| | |
|---|---|
| kp | Siedepunkt |
| $MgSO_4$ | Magnesiumsulfat |
| mp | Schmelzpunkt |
| MS | Massenspektrum |
| NaOH | Natriumhydroxid-Lösung |
| RP | Reversed Phase |
| THF | Tetrahydrofuran |
| TFA | Trifluoressigsäure |

Beispiele:

Wenn nicht anders beschrieben, handelt es sich bei den hier angeführten Beispielen um Racemate.

Die zur Charakterisierung herangezogenen HPLC- bzw. LCMS-Bedingungen waren wie folgt:
HPLC- und HPLC-MSD-Systeme von Agilent Technologies aus der Serie 1100 mit DAD-Detektor, Merck Purospher-Säule (3µ, 2x55mm), Säulentemperatur: 30°C, Wellenlänge: 220 nm, Fluß: 0,5 ml/min, Gradient: von 95% Wasser (0,05% TFA) / 5% Acetonitril in 4 min auf 5% Wasser (0,05% TFA) / 95% Acetonitril, dann 1,5 min bei 5% Wasser (0,05% TFA)/ 95% Acetonitril halten.
Die mit * gekennzeichneten Werte wurden unter folgenden Bedingungen ermittelt:
HPLC-MSD-System von Agilent Technologies aus der Serie 1100 mit DAD-Detektor, Nucleosil-Säule (C-18, 5µ, 4x125 mm), Säulentemperatur: 40°C, Wellenlänge: 220 nm, Fluß: 0,65 ml/min, Gradient: 95% Wasser [Wasser/Acetonitril 9: 1 mit 0,1% TFA] / 5% Acetonitril [Wasser / Acetonitril 1:9 mit 0,1% TFA] für 2 min, dann in 10 min auf 5% Wasser / 95% Acetonitril, dann 5 min bei 5% Wasser / 95% Acetonitril halten.
Zur Charakterisierung der Endverbindungen sind die HPLC-Retentionszeit sowie das Ergebnis der separat durchgeführten massenspektroskopischen Untersuchung angegeben.

Beispiel 1: (rac)-exo/endo-(Octahydro-4,7-methano-inden-5-yl)-pyridin-3-ylmethylamin-Hydrochlorid

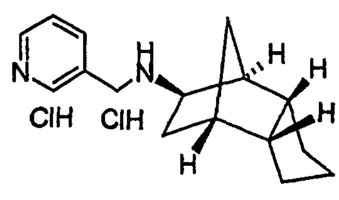

a) exo/endo-3a,4,5,6,7,7a-Hexahydro-1H-4,7-methano-inden-5-ylamin und exo/endo-(3a,4,5,6,7,7a-Hexahydro-3H-4,7-methano-inden-5-yl)-pyridin-3-ylmethyl-amin

10 g exo-5-Isothiocyanat-5,6-dihydroendodicyclopentadien (Maybridge international) wurden in 61 ml Ameisensäure gelöst und 45 Stunden unter Rückfluss gekocht. Nach Abkühlen wurde ein schwarzer Niederschlag abfiltriert

und das Filtrat eingeengt. Der Rückstand wurde mit Wasser verdünnt, und in der Hitze wurden langsam 10 g Natriumhydroxid zugegeben. Danach wurde auf Raumtemperatur abgekühlt, dreimal mit Toluol extrahiert, die vereinigten organischen Phasen mit $MgSO_4$ getrocknet, das $MgSO_4$ abfiltriert und das Filtrat eingeengt. Der Rückstand wurde destilliert und ergab 3,38 g eines klaren Öls.
HPLC-RT=3,15 min; MS (Cl+): 150 $(M+H)^+$.

b) (exo/endo)-Octahydro-4,7-methano-inden-5-ylamin

3,3 g des Doppelbindungsisomerengemisches aus 1a) wurden in 30 ml Methanol gelöst, 0,5 g Palladium auf Kohle (10%) als Katalysator dazugegeben und unter Wasserstoffatmosphäre 4 h hydriert. Anschließend wurde der Katalysator abfiltriert, mit Methanol gewaschen und das Filtrat eingeengt. Nach Trocknen unter Hochvakuum wurden 3 g Produkt als klares Öl erhalten.
HPLC-RT=3,33 min; MS (ES+): 152 $(M+H)^+$

c) exo/endo-(Octahydro-4,7-methano-inden-5-yl)-pyridin-3-ylmethyl-amin-Hydrochlorid

Eine Lösung aus 3 g des exolendo-konfigurierten Octahydro-4,7-methano-inden-5-ylamins aus 1 b) und 2,15 g Pyridin-3-carbaldehyd in 200 ml Toluol wurden nach Zugabe einer katalytischen Menge an p-Toluolsulfonsäure 5 Stunden am Wasserabscheider zum Sieden erhitzt, und nach dem Stehenlassen über Nacht bei Raumtemperatur wurde das Lösungsmittel abdestilliert. Man löste den Rückstand in 150 ml Methanol und gab sodann unter Rühren 0,91 g Natriumboranat in kleinen Portionen zu der eisgekühlten Lösung. Man rührte mehrere Stunden bei Raumtemperatur und stellte sodann mit überschüssiger methanolischer HCl stark sauer. Nach Einengen am Rotationsverdampfer wurde der Rückstand in Wasser gelöst und mit Kaliumcarbonat-Lösung alkalisch gestellt. Anschließend wurde dreimal mit EE extrahiert, die vereinigten Extrakte wurden über Magnesiumsulfat getrocknet, filtriert und mit etherischer HCl auf pH 1 - 2 gestellt. Das Lösungsmittel wurde dann von ausgefallenen Produkt abdekantiert und der Rückstand in Ethanol in der Wärme gelöst. Nach Erkalten wurde das Produkt mit Ether ausgefällt. Es wurden 2,85 g heller Kristalle erhalten.
HPLC-RT=3,15, MS (ES+) : 243,2 $(M+H)^+$

Beispiel 2: (+)-exo/endo-(Octahydro-4,7-methano-inden-5-yl)-pyridin-3-ylmethyl-amin-Hydrochlorid

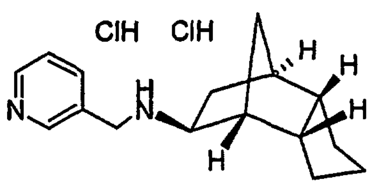

a) (+)-(exo/endo)-Octahydro-4,7-methano-inden-5-ylamin und (-)-(exo/endo)-Octahydro-4,7-methano-inden-5-ylamin

Die Titelverbindungen lassen sich ausgehend vom racemischen (exo/endo)-Octahydro-4,7-methano-inden-5-ylamin aus Beispiel 1 b) entweder über Chromatographie an chiralen Säulen oder durch Kristallisation mit chiralen Säuren enantiomerenrein gewinnen. Unter Verwendung von Z-Valin lässt sich (+)-(exo/endo)-Octahydro-4,7-methano-inden-5-ylamin beispielsweise wie folgt gewinnen:

a1) (+)-(exo/endo)-Octahydro-4,7-methano-inden-5-ylamin durch Racematspaltung mit Z-Valin:

Eine Mischung aus 50 g racemischem (exo/endo)-Octahydro-4,7-methano-inden-5-ylamin aus Beispiel 1b) und 83,2 g Z-Valin in 1,5 l Tetrahydrofuran wurde zum Sieden erhitzt. Die klare Lösung ließ man innerhalb von 3 Stunden auf Umgebungstemperatur abkühlen. Anschließend rührte man noch weitere 20 Stunden bei Umgebungstemperatur. Der Niederschlag wurde abgesaugt, mit 50 ml Tetrahydrofuran gewaschen und bei 40 °C im Trockenschrank getrocknet. Man erhielt 55 g des Salzes mit 65 % Diastereomerenreinheit.
Nach dreimaligem Umkristallisieren aus jeweils einem Liter Tetrahydrofuran erhielt man 30 g des Salzes mit > 95 % Diastereomerenreinheit.

Zu einer Suspension von 9,4 g des obigen Z-Valinsalzes in jeweils 30 ml Toluol und Wasser gab man 1 N Natronlauge, bis der pH-Wert konstant bei 11 blieb. Dabei ging der Feststoff in Lösung. Die Phasen wurden getrennt und die Wasserphase wurde noch dreimal mit jeweils 10 ml Toluol extrahiert. Die vereinigten Toluolphasen wurden über Natriumsulfat getrocknet, und das Toluol wurde im Vakuum abdestilliert.

Das so erhaltene Amin (3,3 g, Enantiomerenreinheit > 95 %) konnte ohne weitere Reinigung umgesetzt werden.

a2) (-)-(exo/endo)-Octahydro-4,7-methano-inden-5-ylamin durch Racematspaltung mit Z-D-Valin:

Das (-)-Enantiomer erhält man analog zur obigen Vorschrift bei Verwendung von Z-D-Valin. So konnten aus 500 mg (+)-(exo/endo)-Octahydro-4,7-methano-inden-5-ylamin aus Beispiel 1b) nach Fällung und zwei Umkristallisationen 468 mg des Salzes mit einer Diastereomerenreinheit > 95 % erhalten werden.

b) (+)-exo/endo-(Octahydro-4,7-methano-inden-5-yl)-pyridin-3-ylmethyl-amin-Hydrochlorid

Eine Lösung aus 1,4 g des (+)-exo/endo-konfigurierten Octahydro-4,7-methano-inden-5-ylamins aus 2a1) und 1 g Pyridin-3-carbaldehyd in 100 ml Toluol wurden nach Zugabe einer katalytischen Menge p-Toluolsulfonsäure 5 Stunden am Wasserabscheider zum Sieden erhitzt, und nach dem Stehenlassen über Nacht bei Raumtemperatur wurde das Lösungsmittel abdestilliert. Man löste den Rückstand in 75 ml Methanol und gab sodann unter Rühren 0,42 g Natriumboranat in kleinen Portionen zu der eisgekühlten Lösung. Man rührte mehrere Stunden bei Raumtemperatur, ließ über Nacht stehen und stellte dann mit überschüssiger methanolischer HCl stark sauer. Die durch Anreiben initiierte Kristallisation ließ man über Nacht im Kühlschrank vervollständigen. Nach Abdekantieren der Lösung wurde der Rückstand mit wässriger Kaliumcarbonat-Lösung aufgenommen und bei pH 11 dreimal mit EE extrahiert, die vereinigten Extrakte wurden über Magnesiumsulfat getrocknet, filtriert und mit methanolischer HCl auf pH 1-2 gestellt. Das Produkt wurde dann durch Zugabe von Ether ausgefällt. Es wurden 1,1 g heller Kristalle erhalten.
HPLC-RT=3,20; MS (Cl+): 243,3 $(M+H)^+$; $[\alpha]_{Na\ 589\ nm}$: +34,6° in Ethanol

Beispiel 3: (-)-exo/endo-(Octahydro-4,7-methano-inden-5-yl)-pyridin-3-ylmethyl-amin-Hydrochlorid

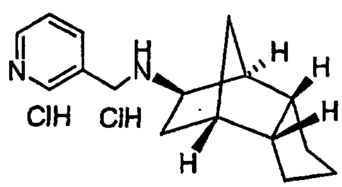

Eine Lösung aus 1,4 g des (-)-exo/endo-konfigurierten Octahydro-4,7-methano-inden-5-ylamins aus 2a2) und 1 g Pyridin-3-carbaldehyd in 100 ml Toluol wurden nach Zugabe einer katalytischen Menge p-Toluolsulfonsäure 5 Stunden am Wasserabscheider zum Sieden erhitzt, und nach dem Stehenlassen über Nacht bei Raumtemperatur wurde das Lösungsmittel abdestilliert. Man löste den Rückstand in 75 ml Methanol und gab sodann unter Rühren 0,42 g Natriumboranat in kleinen Portionen zu der eisgekühlten Lösung. Man rührte mehrere Stunden bei Raumtemperatur, ließ über Nacht stehen und stellte dann mit überschüssiger methanolischer HCl stark sauer. Die durch Anreiben initiierte Kristallisation ließ man über Nacht im Kühlschrank vervollständigen. Nach Abdekantieren der Lösung wurde der Rückstand mit wässriger Kaliumcarbonat-Lösung aufgenommen und bei pH 11 dreimal mit EE extrahiert, die vereinigten Extrakte wurden über Magnesiumsulfat getrocknet, filtriert und mit methanolischer HCl auf pH 1-2 gestellt. Das Produkt wurde dann durch Zugabe von Ether ausgefällt. Es wurden 1,1 g heller Kristalle erhalten.
HPLC-RT=3,12; MS (Cl+): 243,3 $(M+H)^+$; $[\alpha]_{Na\ 589\ nm}$ : -32,5° in Ethanol

Beispiel 4: exo/endo-Furan-2-ylmethyl-(octahydro-4,7-methano-inden-5-yl)-amin-Hydrochlorid

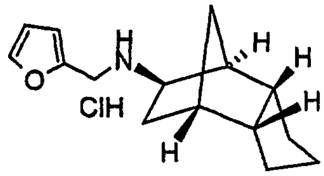

200 mg des exo/endo-konfigurierten Octahydro-4,7-methano-inden-5-ylamins aus Beispiel 1 b), 127 mg 2-Furaldehyd sowie 101 mg p-Toluolsulfonsäure wurden in 20 ml Toluol (wasserfrei) gelöst und 4 Stunden unter Rückfluss gekocht. Nach dem Stehenlassen über Nacht bei Raumtemperatur wurde das Lösungsmittel abdestilliert. Man löste den Rückstand in 15 ml Methanol und gab sodann 0,06 g Natriumboranat in kleinen Portionen unter Rühren zu der eisgekühlten Lösung. Man rührte mehrere Stunden bei Raumtemperatur und stellte sodann mit überschüssiger methanolischer HCl sauer. Nach Einengen am Rotationsverdampfer wurde der Rückstand in 2 N NaOH aufgenommen und dreimal mit EE extrahiert. Die vereinigten organischen Phasen wurden mit methanolischer HCl angesäuert und eingeengt. Der ölige Rückstand wurde mittels präparativer HPLC an RP-18 mit Acetonitril/Wasser (0,05% TFA) gereinigt. Die sauberen Fraktionen wurden vereinigt, das Acetonitril am Rotationsverdampfer entfemt, mit Kaliumcarbonat auf pH 11 gestellt, mit EE extrahiert, die vereinigten EE-Phasen mit $MgSO_4$ getrocknet und nach Abfiltrieren des $MgSO_4$ eingeengt. Der Rückstand wurde mit 2 N Salzsäure aufgenommen und gefriergetrocknet. Es wurden 119 mg des Hydrochlorids als weißer Feststoff erhalten.
HPLC-RT=3,60 min; MS (ES+): 232,2 $(M+H)^+$

Beispiel 5: exo/endo-(Octahydro-4,7-methano-inden-5-yl)-thiophen-2-ylmethyl-amin-Hydrochlorid

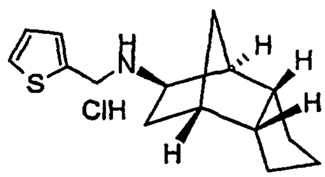

200 mg des exo/endo-konfigurierten Octahydro-4,7-methano-inden-5-ylamins aus Beispiel 1b), 148 mg Thiophen-2-aldehyd sowie 101 mg p-Toluolsulfonsäure wurden in 20 ml Toluol (wasserfrei) gelöst und 4 Stunden unter Rückfluss gekocht. Nach dem Stehenlassen über Nacht bei Raumtemperatur wurde das Lösungsmittel abdestilliert. Man löste den Rückstand in 15 ml Methanol und gab sodann unter Rühren 0,06 g Natriumboranat in kleinen Portionen zu der eisgekühlten Lösung. Man rührte mehrere Stunden bei Raumtemperatur und stellte sodann mit überschüssiger methanolischer HCl sauer. Nach Einengen am Rotationsverdampfer wurde der Rückstand in 2 N NaOH aufgenommen und dreimal mit EE extrahiert. Die vereinigten organischen Phasen wurden mit methanolischer HCl angesäuert und eingeengt. Der ölige Rückstand wurde mittels präparativer HPLC an RP-18 mit Acetonitril/Wasser (0,05% TFA) gereinigt. Die sauberen Fraktionen wurden vereinigt, das Acetonitril am Rotationsverdampfer entfernt, mit Kaliumcarbonat auf pH 11 gestellt, mit EE extrahiert, die vereinigten EE-Phasen mit $MgSO_4$ getrocknet und nach Abfiltrieren des $MgSO_4$ eingeengt. Der Rückstand wurde mit 2 N Salzsäure aufgenommen und gefriergetrocknet. Es wurden 61 mg des Hydrochlorids als weißer Feststoff erhalten. HPLC-RT=3,84 min; MS (Cl+): 248,3 $(M+H)^+$

Beispiel 6: exo/exo-(Octahydro-4,7-methano-inden-5-yl)-pyridin-3-ylmethyl-amin-Hydrochlorid

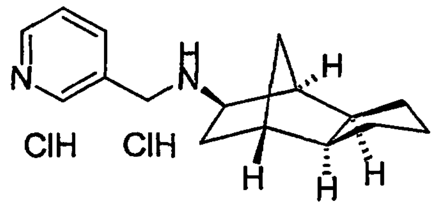

a) Octahydro-4,7-methano-inden-5-ol

25 g Tricyclo[5.2.1.0 (2,6)]decan-8-on (Aldrich) wurden in 100 ml Methanol gelöst und bei Raumtemperatur unter leichter Kühlung und Rühren portionsweise innerhalb von 2 h mit 6,3 g festem Natriumborhydrid versetzt. Anschließend wurde 2 h nachgerührt und über Nacht stehengelassen. Unter Kühlung wurden dann ca. 40 ml 2 N HCl zugetropft, gefolgt von 20 ml Wasser. Das Gemisch wurde eingeengt, der Rückstand mit Essigester versetzt und die Essigesterphase einmal mit Wasser und einmal mit Natriumhydrogencarbonat-Lösung gewaschen. Nach Trocknen mit Magnesiumsulfat wurde die Essigesterphase filtriert und eingeengt. Es blieben 26 g Öl zurück, das durch Vakuumdestillation gereinigt wurde. Es wurden 20,7 g einer öligen Flüssigkeit erhalten ($kp_{0,5}$ 76°C).
HPLC-RT=4,55 min; MS (Cl+): 134,8 $(M-OH)^+$

b) 2-(Octahydro-4,7-methano-inden-5-yl)-isoindol-1,3-dion

Zu einer Lösung aus 1,66 g Octahydro-4,7-methano-inden-5-ol aus 6 a), 1,47 g Phthalimid und 2,62 g Triphenylphosphin in 15 ml THF wurden unter Rühren 1,7 g Diethyl-azodicarboxylat verdünnt mit 5 ml THF gegeben. Nach Stehen über Nacht wurde das Reaktionsgemisch eingedampft, der Rückstand mit Ether verrührt, der Niederschlag abgesaugt und das Filtrat eingeengt. Der Rückstand wurde über Kieselgel/Toluol gereinigt. Es wurden 1,36 g eines gelben Öls erhalten.
HPLC-RT=5,82 min; MS (Cl+): 282,2 $(M+H)^+$

c) exo/exo- Octahydro-4,7-methano-inden-5-ylamin

Zu einer Lösung aus 1,12 g 2-(Octahydro-4,7-methano-inden-5-yl)-isoindol-1,3-dion aus 6 b) und 15 ml Ethanol wurden 0,4 g Hydrazinhydrat getropft und 2 h bei 65 °C gerührt. Anschließend wurde mit konz. HCl auf pH 1 - 2 gestellt, mit 10 ml Ethanol versetzt, der Niederschlag abfiltriert und das Filtrat eingeengt. Der Rückstand wurde mittels präparativer HPLC an RP-18 mit Acetonitril/Wasser (0,05% Trifluoressigsäure) gereinigt. Nach Gefriertrocknung wurden 567 mg Produkt als Trifluoracetat erhalten. Behandlung mit Natronlauge und Essigester lieferte 322 mg des freien Amins.
HPLC-RT=3,47 min; MS (Cl+): 152,0 $(M+H)^+$

d) exo/exo-(Octahydro-4,7-methano-inden-5-yl)-pyhdin-3-ylmethyl-amin-Hydrochlorid

Eine Lösung aus 332 mg des exo/exo-konfigurierten Octahydro-4,7-methano-inden-5-ylamins aus 6 c) und 215 mg Pyridin-3-carbaldehyd in 20 ml Toluol wurden nach Zugabe einer katalytischen Menge p-Toluolsulfonsäure 5 Stunden zum Rückfluss erhitzt, und nach dem Stehenlassen über Nacht bei Raumtemperatur wurde das Lösungsmittel abdestilliert. Man löste den Rückstand in 15 ml Methanol und gab sodann unter Rühren 91 mg Natriumboranat in kleinen Portionen zu der gekühlten Lösung. Man rührte mehrere Stunden bei Raumtemperatur und stellte dann mit überschüssiger methanolischer HCl stark sauer. Nach Einengen am Rotationsverdampfer wurde der Rückstand mit 2 N Natriumhydroxid-Lösung aufgenommen. Nach dreimaligem Extrahieren mit EE wurden die vereinigten Extrakte eingeengt, und der Rückstand wurde mittels präparativer HPLC an RP-18 mit Acetonitril/Wasser (0,05% TFA) gereinigt. Die Produkt-enthaltenden Fraktionen wurden vereinigt, gefriergetrocknet und erneut per HPLC gereinigt. Die sauberen Fraktionen wurden vereinigt, das Acetonitril am Rotationsverdampfer entfernt, mit Kaliumcarbonat auf pH 11 gestellt, mit EE extrahiert, die vereinigten EE-Phasen mit $MgSO_4$ getrocknet und nach Abfiltrieren des $MgSO_4$ eingeengt. Der Rückstand wurde mit 2 N Salzsäure aufgenommen und gefriergetrocknet. Es wurden 35 mg des Hydrochlorids als weißer Feststoff erhalten.

HPLC-RT=3,25 min, MS (ES+): 243,1 $(M+H)^+$

Beispiel 7: exo/endo-(3-Chloro-5-trifluoromethyl-pyridin-2-ylmethyl)-(octahydro-4,7-methano-inden-5-yl)-amin-Trifluoresslgsäuresalz

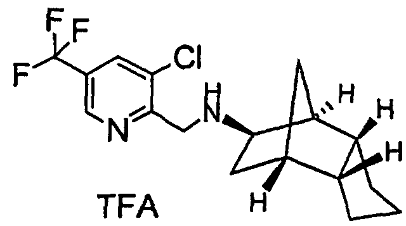

0,5.mmol exo/endo-Octahydro-4,7-methano-inden-5-ylamin aus Beispiel 1 b), 0,5 mmol 3-Chloro-5-trifluoromethyl-pyridin-2-carbaldehyd, 140 µl Triethylamin und 10 ml Dichlormethan wurden vorgelegt, 250 µl einer 1-molaren Lösung von Titantetrachlorid in Toluol zugetropft und 24 h bei Raumtemperatur gerührt. Anschließend wurden 1,5 ml einer 1-molaren Lösung von Natriumcyanoborhydrid in THF langsam zugegeben und 30 min bei Raumtemperatur gerührt. Dann wurde mit 15 ml 2 N NaOH versetzt und 15 min gerührt. Es wurde abfiltriert und mit Wasser gewaschen. Zum Filtrat wurden 30 ml EE gegeben, geschüttelt und dann die organische Phase abgetrennt. Nach Trocknen wurde eingeengt und der Rückstand mittels präparativer HPLC gereinigt (RP18, Gradient Acetonitril/Wasser 30% -> 90%, mit 0.1 % TFA in beiden Komponenten). Nach Gefriertrocknung wurden 4,7 mg als weißer Feststoff erhalten.
HPLC-RT= 11,23 min*, MS (ES+): 345,2 $(M+H)^+$

Beispiel 8: exo/endo-(Decahydro-1,4-methano-naphthalen-2-yl)-pyridin-3-ylmethylamin-Hvdrochlorid

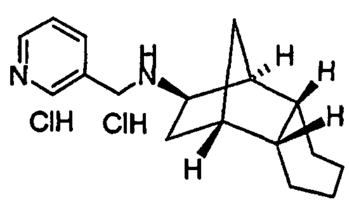

a) Bis-(3-chloro-1,2,3,4-tetrahydro-1,4-methano-naphthalen-2-yl)-diazen N,N'-dioxid

Zu einer Lösung von 3,56 g Benzonorbomadien [L.Friedman and F.M.Logullo, J. Org. Chem. 34: 3089-3092, (1969)] in 6 ml Eisessig und 6 ml Ethanol gab man 3,34 g iso-Amylnitrit und tropfte sodann 8,5 ml einer 15%igen Lösung von Chlorwasserstoffgas in Ethanol zu. Die entstehende Suspension wurde weitere 2 ½ Stunden bei Raumtemperatur gerührt und anschließend mit 20 mg Diisopropylether versetzt. Nach weiterer Rührung über 30 Minuten filtrierte man den Feststoff ab. Heller kristalliner Feststoff; mp. 187 - 188°C
MS (FAB): 415,1 $(M+H)^+$

b) (exo)-1,2,3,4-Tetrahydro-1,4-methano-naphthalen-2-ylamin

3 g Bis-(3-chloro-1,2,3,4-tetrahydro-1,4-methano-naphthalen-2-yl)-diazen N,N'- dioxid wurden in 150 ml Methanol suspendiert und mit Raney-Nickel Katalysator im Autoklaven mit Wasserstoff bei 100 bar und 100°C 20 Stunden hydriert. Nach dem Abfiltrieren des Katalysators verdampfte man das Lösungsmittel, versetzte den Rückstand mit Wasser, stellte mit NaOH stark alkalisch und extrahierte mehrfach mit Methyl-tert.butylether. Nach Trocknung der organischen Phasen erhielt man das gewünschte Amin als hellgelbe Flüssigkeit.
MS (ES+): 160,0 $(M+H)^+$

c) exo/endo-Decahydro-1,4-methano-naphthalen-2-ylamin

Eine Lösung von 1 g exo/endo-1,2,3,4-Tetrahydro-1,4-methano-naphthalen-2-ylamin in 10 ml Methanol und 30 ml 2 N Salzsäure wurde mit 0,4 g $RuO_2$ mit Wasserstoff bei 100 bar und 90°C 10 Stunden im Autoklaven hydriert. Nach Abtrennen des Katalysators wurde auf die Hälfte eingedampft, die so erhaltene wässrige Lösung mit 10 N NaOH stark alkalisch gestellt und mehrfach mit Methyl-tert.butylether extrahiert. Nach dem Trocknen und Verdampfen des Lösungsmittels erhielt man exo/endo-Decahydro-1,4-methano-naphthalen-2-ylamin als farbloses Öl, das vorzugsweise unter Argon gelagert wurde.
MS (Cl+): 166,2 $(M+H)^+$

d) exo/endo-(Decahydro-1,4-methano-naphthalen-2-yl)-pyridin-3-ylmethyl-amin-Hydrochlorid

Eine Lösung aus 0,71 g Pyridin-3-aldehyd und 1,1 g exo/endo-Decahydro-1,4-methano-naphthalen-2-ylamin in 40 ml Toluol wurde nach Zugabe einer kleinen katalytischen Menge an p-Toluolsulfonsäure (3-5 mg) 4 Stunden unter Rückfluss gekocht und sodann das Lösungsmittel abdestilliert. Nach Auflösen des öligen Rückstands in ca. 30 ml wasserfreiem Methanol versetzte man unter Kühlung und Rührung portionsweise mit 0,335g Natriumborhydrid, ließ weitere 2 Stunden bei Raumtemperatur rühren und über Nacht stehen. Dann stellte man mit einer Lösung von HCl in Methanol sauer, filtrierte den Niederschlag ab und verdampfte das Lösungsmittel. Der Rückstand wurde aus Ethanol umkristallisiert, mp. 283-285°C.
HPLC-RT=3,38 min, MS (Cl+): 257,4 $(M+H)^+$

Die nachfolgend beschriebenen Verbindungen wurden aus literaturbekannten Carbonylderivaten und den entsprechenden Aminen analog des angegebenen Beispiels dargestellt:

| Beispiel | | Salz | Analog Beispiel | MS | HPLC-RT [min] |
|---|---|---|---|---|---|
| 9 | 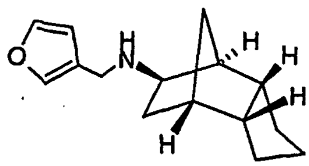 | HCl | 5 | ES+ 232,2 (M+H)+ | 3,73 |
| 10 | 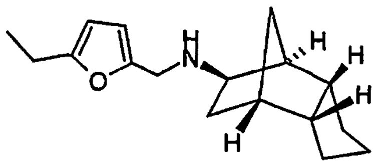 | HCl | 5 | ES+ 260,2 (M+H)+ | 4,00 |
| 11 | 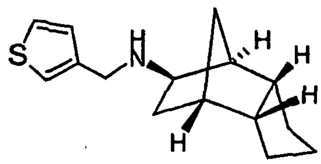 | HCl | 5 | CI+ 248,0 (M+H)+ | 3,88 |
| 12 | 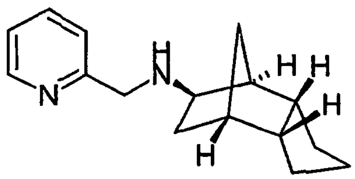 | HCl | 5 | ES+ 243,1 (M+H)+ | 3,63 |
| 13 | 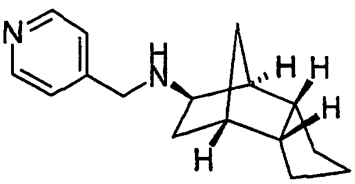 | HCl | 5 | CI+ 243,0 (M+H)+ | 3,14 |
| 14 | 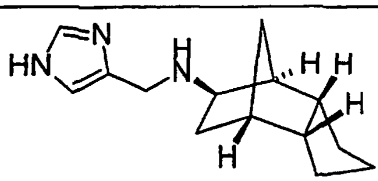 | HCl | 5 | CI+ 232,2 (M+H)+ | 3,05 |
| 15 | 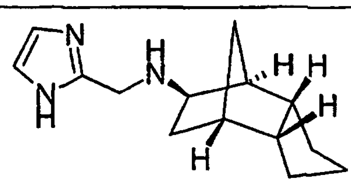 | HCl | 5 | CI+ 232,1 (M+H)+ | 3,05 |

| | | | | | |
|---|---|---|---|---|---|
| 16 | 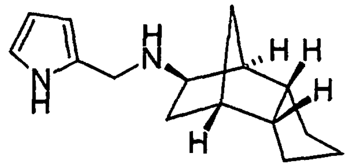 | HCl | 5 | ES+ 231,2 (M+H)+ | 3,76 |
| 17 | 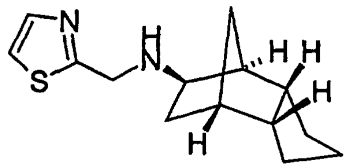 | HCl | 5 | ES+ 249,1 (M+H)+ | 3,47 |
| 18 (Racemat) | 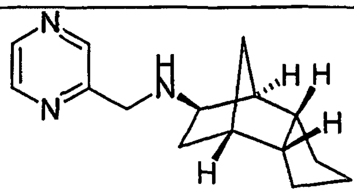 | HCl | 5 | Cl+ 244,1 (M+H)+ | 3,39 |
| 19 (+) | 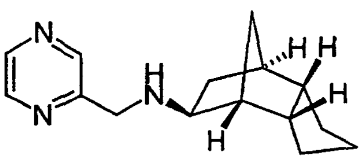 | HCl | 2 | Cl+ 244,2 (M+H)+ | 3,41 |
| 20 (-) | 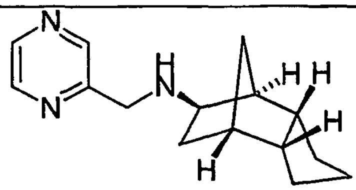 | HCl | 3 | Cl+ 244,2 (M+H)+ | 3,40 |
| 21 | 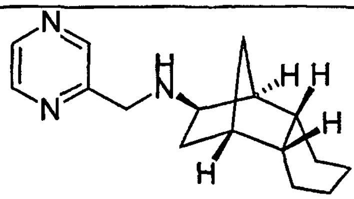 | HCl | 8 | ES+ 258,1 (M+H)+ | 3,65 |
| 22 | 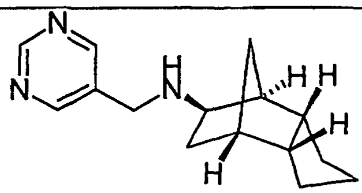 | HCl | 5 | ES+ 244,1 (M+H)+ | 3,33 |

| | | | | | |
|---|---|---|---|---|---|
| 23 | 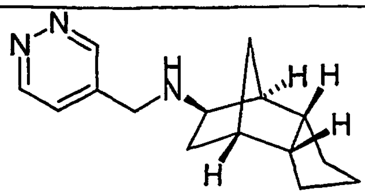 | HCl | 5 | ES+ 244,2 (M+H)+ | 3,43 |
| 24 | 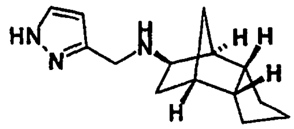 | HCl | 5 | CI+ 232,1 (M+H)+ | 3,42 |
| 25 | 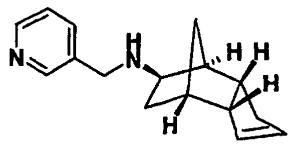 Gemisch 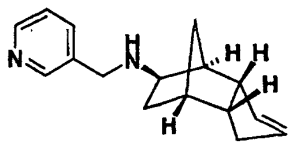 | HCl | 5 | CI+ 241,2 (M+H)+ | 3,07 |
| 26 | 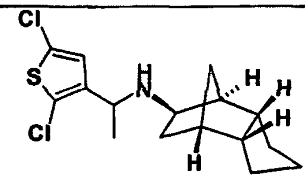 | TFA | 7 | ES+ 330,1 (M+H)+ | 11,78* |
| 27 | 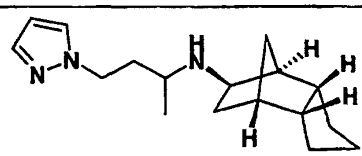 | TFA | 7 | ES+ 274,2 (M+H)+ | 9,75* |
| 28 | 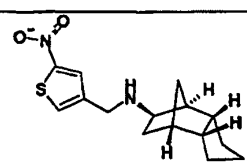 | TFA | 7 | ES+ 293,1 (M+H)+ | 10,03* |
| 29 | 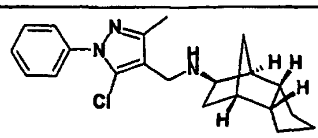 | TFA | 7 | ES+ 356,3 (M+H)+ | 11,18* |
| 30 | 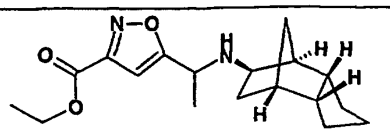 | TFA | 7 | ES+ 319,3 (M+H)+ | 9,99* |

Pharmakologische Daten:

Beschreibung des Caco 2-Modells

Die Caco-2-Zellinie wurde bei American Type Culture Collection (ATCC) erworben und in Dulbecco's Modified Eagle Medium (hoher Glucoseanteil), ergänzt mit nichtessenziellen Aminosäuren, L-Glutamin, Penicillin/Streptomycin und 10 %igem fötalen Kälberserum, in einem Inkubator unter einer 10%igen $CO_2$-Atmosphäre bei 95%iger relativer Luftfeuchtigkeit und 37°C gehalten. Die Zellen wurden in Zellkulturkolben (175 $cm^2$) gezogen.
Für die Transport-Untersuchungen wurden die Caco-2-Zellen auf Polycarbonat Zellkultureinlagen (Costar Transwells®, Porengröße: 3 µm, Fläche: 4,71 $cm^2$) mit einer Zelldichte von 6,5 x $10^4$ Zellen/$cm^2$ ausgesät und in Sechs-Well-Kulturplatten mit Mediumwechsel nach vier und acht Tagen und dann jeden zweiten Tag inkubiert. Für die Experimente wurden 21 bis 25 Tage alte Monoschichten verwandt.

In jeder Testreihe wurde eine 21 Tage alte Monoschicht mit $^3$H-Dextran als Permeabilitätsmarker auf ihre Eigenschaften getestet. Der Wert der Transferrate (kumulativ) musste nach 120 min im Bereich von 2 % sein.

Nach Beseitigen des Wachstummediums von der apicalen und der basolateralen Seite wurden die Monoschichten mit dem Transport-Puffer (Hank's balanced salt solution pH 7,8; enthält 2,8 g/l Glucose) gespült, und die Zellen wurden 15 min bei 37°C unter 10%iger $CO_2$-Atmosphäre equilibriert. Danach wird der HBSS-Puffer wieder entfernt. Die Testverbindungen wurden in einem Gemisch aus HBSS-Puffer und DMSO gelöst und zum apicalen Puffer gegeben, so dass eine 1 %ige (V/V) DMSO-Lösung resultierte. Die Testkonzentration im ersten Versuch betrug 1 mM, im zweiten 100µM. Die Versuche wurden bei 37°C durchgeführt und mit der Zugabe von 1,5 ml Testlösung auf der Donorseite (apical) gestartet. Transport-Puffer ohne Verbindung wurde auf die Empfängerseite (basolateral, 2,5 ml) gegeben. Zu verschiedenen Zeitpunkten wurden Proben von der basolateralen Seite genommen (1 ml) und durch frische 37°C warme Pufferlösung ersetzt: Apicale Proben wurden zu Beginn und am Ende (120 min) genommen, um anhand dieser Konzentrationen und der kumulativen basolateralen Konzentration die Wiederfindungsrate der Verbindungen zu bestimmen. Die Verbindungen wurden mittels HPLC analysiert.
Der scheinbare Permeabilitätskoeffizient ($P_{app}$) wird über folgende Gleichung berechnet:

$$P_{app} = \frac{d_c \cdot V}{d_t \cdot A \cdot c_0}$$

darin bedeuten $d_c/d_t$ den Fluss durch die Monoschicht (µg oder Verbindung/ml x s), V das Flüssigkeitsvolumen in der Auffangkammer (ml), A die Oberflächengröße der Monoschicht ($cm^2$) und $c_0$ die Anfangskonzentration (µg oder Verbindung/ml) in der Donorkammer. Der Fluss durch die Monoschicht wurde aus der kumulativen basolateralen Konzentration zum entsprechende Zeitpunktpunkt unter Zuhilfenahme der anfänglich linearen Datenkurve (linear bis zu 60 min) errechnet. Die jeweiligen Bestimmungen wurden dreifach gemacht, so dass der berechnete $P_{app}$-Wert den Mittelwert dreier Messungen darstellt. $P_{app}$-Werte ausgewählter Verbindungen wurden mit literaturbekannten Absorptions-Werten korreliert und ergaben eine sigmoidale Kalibrierungskurve. Nach Untersuchungen von Artursson (Artursson P., Karlsson J.; Biochem. Biophys. Res. Comm. 1991;175/3: 880 - 885) lässt sich anhand dieser Kurve eine Aussage über den absorbierten Anteil einer Verbindung machen.

Ergebnisse:

| | | absorbierter Anteil [%] |
|---|---|---|
| Beispiel 1 | 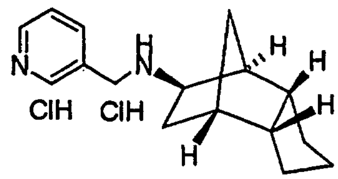 | 100 |
| Beispiel 18 | 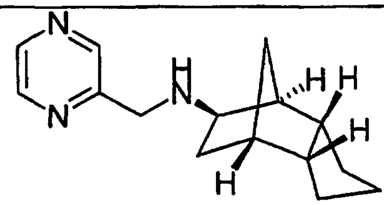 | 100 |
| S 3226 | 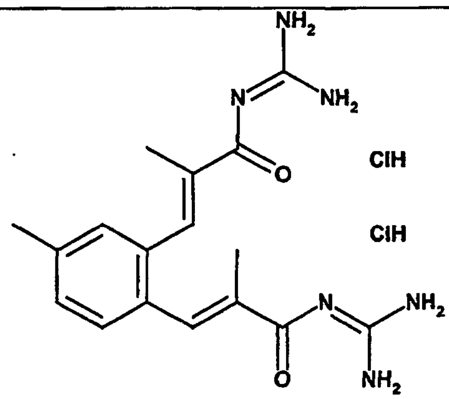 | <5 |
| S 2120 | 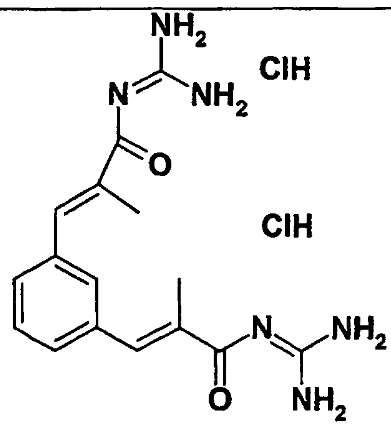 | <1 |

Gegenüber den literaturbekannten NHE3-aktiven Verbindungen vom Acylguanidin-Typ (J.-R. Schwark et al. Eur. J. Physiol (1998) 436:797) zeigen die Verbindungen der Formel I oder I a eine deutlich überlegene Membrangängigkeit.

Beschreibung der NHE-Aktivitätsmessungen:

Die meisten der molekularbiologischen Techniken folgen Protokollen aus den Werken "Current Protocols in Molecular Biology (eds. Ausubel, F.M., Brent, R., Kingston, R.E., Moore, D.D., Seidman, J.G., Smith, J.A. und Struhl, K.; John Wiley & Sons)" bzw: "Molecular Cloning: A Laboratory Manual (Sambrock, J., Fritsch, E.F. und Maniatis, T.; Cold Spring Harbor Laboratory Press (1989))".
Im Rahmen unserer Arbeiten wurden stabil transfizierte Zellinien erzeugt, die jeweils einen der folgenden NHE-Subtypen exprimieren: NHE1 des Menschen (Sardet et al. (1989) Cell 56, 271-280), NHE2 des Kaninchens (Tse et al. (1993) J. Biol. Chem. 268, 11917 -11924), NHE3 des Menschen (Brant et al. (1995) Am. J. Physiol. 269 (Cell Physiol. 38), C198 - C206) bzw. NHE3 der Ratte (Orlowski et al.; J. Biol. Chem. 267, 9331 - 9339 (1992)).
Die von Prof. Pouysségur erhaltenen cDNA-Klone der jeweiligen NHE-Subtypen wurden nach Anfügen geeigneter Linkersequenzen so in das Expressionsplasmid pMAMneo (erhältlich z. B. über CLONTECH, Heidelberg) einkloniert, dass die Erkennungssequenz für die Restriktionsendonuklease NheI des Plasmids etwa 20 - 100 Basenpaare vor dem Startcodon des jeweiligen NHE-Subtyps liegt und die gesamte codierende Sequenz in dem Konstrukt vorhanden ist. Bei dem über RT-PCR aus humaner Nieren mRNA erhaltenem humanen NHE3 wurden die RT-PCR Primer so gewählt, dass die erhaltene cDNA-Bande an ihren Enden zu pMAMneo passende Schnittstellen aufweist.
Mit der sog. "Calciumphosphat-Methode" (beschrieben in Kapitel 9.1 von "Current Protocols in Molecular Biology") wurde die NHE-defiziente Zellinie LAP1 (Franchi et al.; Proc. Natl. Acad. Sci. USA 83, 9388 - 9392 (1986)) mit den Plasmiden, die die jeweiligen codierenden Sequenzen der NHE-Subtypen erhalten, transfiziert. Nach Selektion auf transfizierte Zellen über Wachstum in G418-haltigem Medium (nur Zellen, die durch Transfektion ein neo-Gen erhalten haben, können unter diesen Bedingungen überleben) wurde auf eine funktionelle NHE-Expression selektioniert. Dazu wurde die von Sardet beschriebene "Acid Load"-Technik verwendet (Sardet et al.; Cell 56, 271 - 280 (1989)). Zellen, die einen funktionsfähigen NHE-Subtypen exprimieren, können auch bei Abwesenheit von $CO_2$ und $HCO_3^-$ die bei diesem Test vorgenommene Ansäuerung kompensieren, untransfizierte LAP1-Zellen dagegen nicht. Nach mehrmaliger Wiederholung der "Acid Load" Selektion wurden die überlebenden Zellen in Mikrotiterplatten so ausgesät, dass statistisch eine Zelle pro Well vorkommen sollte. Unter dem Mikroskop wurde nach etwa 10 Tagen überprüft, wie viele Kolonien pro Well wuchsen. Zellpopulationen aus Einzelkolonien wurden dann mit dem XTT-Proliferation Kit (Boehringer Mannheim) hinsichtlich ihrer Überlebensfähigkeit nach "Acid Load" untersucht. Die besten Zellinien wurden für die weiteren Tests verwendet und zur Vermeidung eines Verlustes der transfizierten Sequenz unter ständigem Selektionsdruck in G418-haltigem Medium kultiviert. Zur Bestimmung von $IC_{50}$-Werten für die Hemmung der einzelnen NHE-Subtypen durch spezifische Substanzen wurde ein von S. Faber entwickelter Test (Faber et al.; Cell. Physiol. Biochem. 6, 39 - 49 (1996)), der auf der "Acid Load" Technik beruht, leicht abgewandelt.
In diesem Test wurde die Erholung des intrazellulären pHs ($pH_i$) nach einer Ansäuerung ermittelt, die bei funktionsfähigem NHE auch unter bicarbonatfreien Bedingungen einsetzt. Dazu wurde der $pH_i$ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den $pH_i$ umgerechnet. Abweichend von dem beschriebenen Protokoll wurden die Zellen bereits bei der BCECF-Beladung in $NH_4Cl$-Puffer (pH 7,4) inkubiert ($NH_4Cl$-Puffer: 115 mM NaCl, 20 mM $NH_4Cl$, 5 mM KCl, 1 mM $CaCl_2$, 1 mM $MgSO_4$, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines $NH_4Cl$-freien Puffers zu 25 µl Aliquots der in $NH_4Cl$-Puffer inkubierten Zellen induziert. Die nachfolgende Geschwindigkeit der pH-Erholung wurde bei NHE1 2 Minuten, bei NHE2 5 Minuten und bei NHE3 3 Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in $Na^+$-haltigem Puffer inkubiert (133,8 mM NaCl, 4,7 mM KCl, 1,25 mM $CaCl_2$, 1,25 mM $MgCl_2$, 0,97 mM $Na_2HPO_4$, 0,23 mM $NaH_2PO_4$, 5 mM Hepes, 5 mM Glucose, mit 1 M NaOH wird ein pH von 7,0 eingestellt). Für die Bestimmung des 0%-Wertes wurden die Zellen in einem $Na^+$- freien Puffer inkubiert (133,8 mM Cholinchlorid, 4,7 mM KCl, 1,25 mM $CaCl_2$, 1,25 mM $MgCl_2$, 0,97 mM $K_2HPO_4$, 0,23 mM $KH_2PO_4$, 5 mM Hepes, 5 mM Glucose, mit 1 M NaOH wird ein pH von 7,0 eingestellt). Die zu testenden Substanzen wurden in dem $Na^+$-haltigem Puffer angesetzt. Die Erholung des intrazellulären pHs bei jeder getesteten Konzentration einer Substanz wurde in Prozent der maximalen Erholung ausgedrückt. Aus den Prozentwerten der pH-Erholung wurde mittels des Programms SigmaPlot der $IC_{50}$-Wert der jeweiligen Substanz für die einzelnen NHE-Subtypen berechnet.

| NHE-Aktivität | |
|---|---|
| Beispiel | Ratten-NHE3 $IC_{50}$ [μM] |
| 1 | 0,34 |
| 5 | 2,9 |
| 6 | 2,1 |

**Patentansprüche**

**1.** Substituierte Heterocyclo-Norbomylamino-Derivate mit exo-konfiguriertem Stickstoff und endo-anelliertem Fünf- oder Sechsring der Formel I oder mit exo-konfiguriertem Stickstoff und exo-anelliertem Fünf- oder Sechsring der Formel I a,

I.

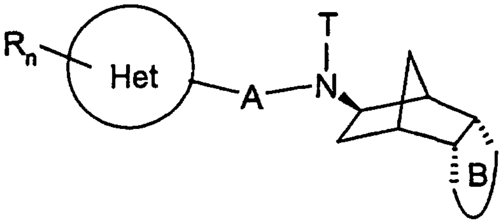

I a

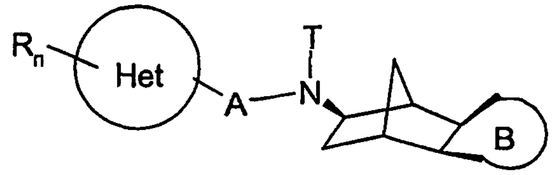

worin bedeuten:

A ($C_1$-$C_4$)- Alkylen;
T ($C_1$-$C_4$)- Alkyl oder H;
B einen gesättigten oder ungesättigten Kohlenstoff-Fünf- oder Sechsring, der unsubstituiert ist oder substituiert ist mit 1 - 3 Substituenten ausgewählt aus der Gruppe bestehend aus Oxo, Hydroxy, ($C_1$-$C_4$)- Alkoxy und ($C_1$-$C_4$)- Alkyl;
Het einen 5- oder 6-gliedrigen, gesättigten oder ungesättigten Heterocyclus, der bis zu vier gleiche oder verschiedene Heteroatome ausgewählt aus der Gruppe bestehend aus O, S, N und Se enthält;
R OH, F, Cl, Br, I, CN, $NO_2$, Phenyl, $CO_2$R1, ($C_1$-$C_4$)- Alkyl, ($C_1$-$C_4$)- Alkoxy, Amino, ($C_1$-$C_4$)- Alkylamino, Di-($C_1$-$C_4$)- alkylamino, Amino-($C_1$-$C_4$)- alkyl,
wobei die Alkylreste unsubstituiert sind oder ganz oder teilweise durch Fluor substituiert sind;

R1 H oder ($C_1$-$C_4$)-Alkyl, das unsubstituiert oder ganz oder teilweise durch Fluor substituiert ist;

n 0, 1, 2, 3 oder 4,
wobei, wenn n = 2, 3 oder 4 ist, die Substituenten R unabhängig voneinander sind;

sowie deren pharmazeutisch verträgliche Salze oder Trifluoracetate.

**2.** Verbindungen mit exo-konfiguriertem Stickstoff und endo-anelliertem Kohlenstoff-Fünf- oder Sechsring der Formel I oder mit exo-konfiguriertem Stickstoff und exo-anelliertem Kohlenstoff Fünf- oder Sechs ring der Formel I a nach Anspruch 1, **dadurch gekennzeichnet, dass** darin bedeuten:

A ($C_1$-$C_2$)- Alkylen;
T H oder Methyl;
B einen gesättigten oder ungesättigten Kohlenstoff-Fünf- oder Sechsring;
Het einen 5- oder 6-gliedrigen, gesättigten oder ungesättigten Heterocyclus, der bis zu drei gleiche oder verschiedene Heteroatome ausgewählt aus der Gruppe bestehend aus O, S und N enthält.;
R F, Cl, Br, Iod, Amino, Hydroxymethyl, OH, Phenyl, $CO_2$R1, ($C_1$-$C_4$)- Alkyl oder ($C_1$-$C_4$)- Alkoxy,

wobei die Alkylreste unsubstituiert oder ganz oder teilweise durch Fluor substituiert sind;

R1 H oder ($C_1$-$C_4$)-Alkyl, wobei der Alkylrest unsubstituiert oder ganz oder teilweise durch Fluor substituiert ist;

n 0, 1, 2 oder 3,
wobei, wenn n = 2 oder 3 ist, die entsprechenden Substituenten R unabhängig voneinander sind;

sowie deren pharmazeutisch verträgliche Salze oder Trifluoracetate.

**3.** Verbindungen mit exo-konfiguriertem Stickstoff und endo-anelliertem Kohlenstoff-Fünf- oder Sechsring der Formel I oder mit exo-konfiguriertem Stickstoff und exo-anelliertem Kohlenstoff-Fünf- oder Sechsring der Formel I a nach Ansprüchen 1 oder 2, **dadurch gekennzeichnet, dass** darin bedeuten:

A ($C_1$-$C_2$)- Alkylen;
T Wasserstoff;
B einen gesättigten oder ungesättigten Kohlenstoff-Fünf- oder Sechsring;
Het einen 5- oder 6-gliedrigen, gesättigten oder ungesättigten Heterocyclus, der bis zu zwei gleiche oder verschiedene Heteroatome ausgewählt aus der Gruppe bestehend aus O, S und N enthält ;
R F, Cl, Br, ($C_1$-$C_4$)- Alkoxy oder ($C_1$-$C_4$)- Alkyl,
wobei die Alkylreste unsubstituiert sind oder ganz oder teilweise durch Fluor substituiert;
n 0, 1 oder 2, wobei, wenn n = 2, die entsprechenden Substituenten R unabhängig voneinander sind;

sowie deren pharmazeutisch verträgliche Salze oder Trifluoracetate.

**4.** Verbindungen mit exo-konfiguriertem Stickstoff und endo-anelliertem Kohlenstoff-Fünf- oder Sechsring der Formel I oder mit exo-konfiguriertem Stickstoff und exo-anelliertem Kohlenstoff-Fünfring der Formel I a nach Anspruch 1, **dadurch gekennzeichnet, dass** sie sind:

exo/exo-{Octahydro-4,7-methano-inden-5-yl)-pyridin-3-ylmethyl-amin,
(rac)-exo/endo-(Octahydro-4,7-methano-inden-5-yl)-pyridin-3-ylmethyl-amin,
(+)-exo/endo-(Octahydro-4,7-methano-inden-5-yl)-pyridin-3-ylmethyl-amin,
(-)-exo/endo-(Octahydro-4,7-methano-inden-5-yl)pyridin-3-ylmethyl-amin,
(rac)-exolendo-(Octahydro-4,7-methano-inden-5-yl)-pyrazin-2-ylmethyl-amin,
(+)-exo/endo-(Octahydro-4,7-methano-inden-5-yl)-pyrazin-2-ylmethyl-amin,
(-)-exo/endo-(Octahydro-4,7-methano-inden-5-yl)pyrazin-2-ylmethyl-amin,
exo/endo-(Decahydro-1,4-methano-naphthalen-2-yl)-pyrazin-2-ylmethyl-amin,
exo/endo-(Octahydro-4,7-methano-inden-5-yl)-thiophen-2-ylmethyl-amin,
exo/endo-(Octahydro-4,7-methano-inden-5-yl)-thiophen-3-ylmethyl-amin,
exo/endo-(3a,4,5,6,7,7a-Hexahydro-3H-4,7-methano-inden-5-yl)-pyridin-3-yl-methyl-amin,
exo/endo-(3a,4,5,6,7,7a-Hexahydro-1H-4,7-methano-inden-5-yl)-pyridin-3-yl-methylamin,
exo/endo-Furan-3-ylmethyl-(octahydro-4,7-methano-inden-5-yl)-amin,
exo/endo-Furan-2-ylmethyl-(octahydro-4,7-methano-inden-5-yl)-amin,
exo/endo-(Decahydro-1,4-methano-naphthalen-2-yl)-pyridin-3-ylmethyl-amin,
exo/endo-(Octahydro-4,7-methano-inden-5-yl)-(1H-pyrrol-2-ylmethyl)-amin,
exo/endo-(Octahydro-4,7-methano-inden-5-yl)-pyrimidin-5-ylmethyl-amin

sowie deren pharmazeutisch verträgliche Salze oder Trifluoracetate.

**5.** Verbindungen mit exo-konfiguriertem Stickstoff und endo-anelliertem Kohlenstoff-Fünf- oder Sechsring der Formel I oder mit exo-konfiguriertem Stickstoff und exo-anelliertem Kohienstoff-Fünfring der Formel I a nach Anspruch 1, **dadurch gekennzeichnet, dass** sie sind:

exo/exo-(Octahydro-4,7-methano-inden-5-yl)-pyridin-3-ylmethyl-amin,
(rac)-exo/endo-(Octahydro-4,7-methano-inden-5-yl)-pyridin-3-ylmethyl-amin,
(+)-exo/endo-(Octahydro-4,7-methano-inden-5-yl)-pyridin-3-ylmethyl-amin,
(-)-exo/endo-(Octahydro-4,7-methano-inden-5-yl)-pyridin-3-ylmethyl-amin,
(rac)-exo/endo-(Octahydro-4,7-methano-inden-5-yl)-pyrazin-2-ylmethyl-amin,

(+)-exo/endo-(Octahydro-4,7-methano-inden-5-yl)-pyrazin-2-ylmethyl-amin,
exo/endo-(Octahydro-4,7-methano-inden-5-yl)-thiophen-2-ylmethyl-amin,
exo/endo-(3a,4,5,6,7,7a-Hexahydro-3H-4,7-methano-inden-5-yl)-pyridin-3-yl-methyl-amin,
exo/endo-(3a,4,5,6,7,7a-Hexahydro-1H-4,7-methano-inden-5-yl)-pyridin-3-yl-methylamin,
exo/endo-(Decahydro-1,4-methano-naphthalen-2-yl)-pyridin-3-ylmethyl-amin,
exo/endo-(Octahydro-4,7-methano-inden-5-yl)-(1H-pyrrol-2-ylmethyl)-amin,
exo/endo-(Octahydro-4,7-methano-inden-5-yl)-pyrimidin-5-ylmethyl-amin

sowie deren pharmazeutisch verträgliche Salze oder Trifluoracetate.

6. Verfahren zur Herstellung der Verbindungen der Formel I oder I a nach Anspruch 1, **dadurch gekennzeichnet, dass** man

a) eine Verbindung der Formel II oder II a

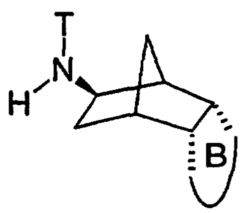
II

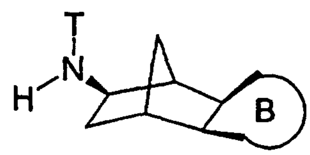
II a

mit einer Verbindung der Formel III

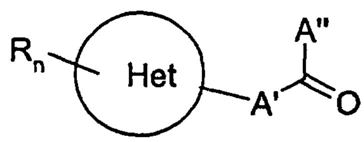
III

in Gegenwart geeigneter Reduktionsmittel und möglicherweise auch Lewis-Säuren direkt zu Verbindungen der Formel I oder I a umsetzt,
worin T, B, Het und $R_n$ die oben angegebene Bedeutung besitzen, während unabhängig voneinander A' einer Bindung oder ($C_1$-$C_3$)- Alkyl und A" H oder ($C_1$-$C_3$)-Alkyl entspricht und A' und A" zusammen mit dem Kohlenstoffatom der Carbonylgruppe so viele Kohlenstoffatome repräsentieren wie das oben beschriebene A;
oder
b) das aus Verbindungen der Formeln II oder II a und III gebildete Intermediat der Formel IV oder IV a,
c)

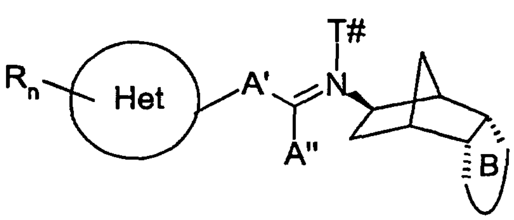
IV

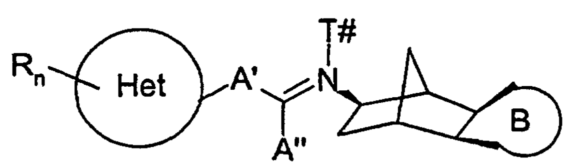
IV a

worin T# einem freien Elektronenpaar oder ($C_1$-$C_4$)-Alkyl entspricht und bei letzterer Bedeutung ein Onium-Stickstoff gebildet wird, dem ein Gegenion, wie Chlorid oder Tosylat, zugeordnet ist,
isoliert und dann mit geeigneten Reduktionsmitteln in die Verbindungen der Formel I oder I a überführt,
oder
c) eine Verbindung der Formel II oder II a mit einem Alkylierungsmittel der Formel V,

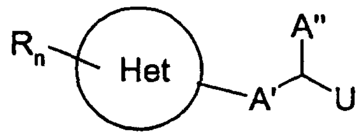

V

in der U für eine nukleophil substituierbare Gruppe steht - wie z. B. Halogen, Alkyloder Arylsulfonate - und die anderen Reste wie oben beschrieben definiert sind, aber hier dem Kohlenstoffatom der Carbonylgruppe das Kohlenstoffatom, an das U gebunden ist, entspricht,
umsetzt
oder
d) Carbonsäureamide der Formel VI oder VI a

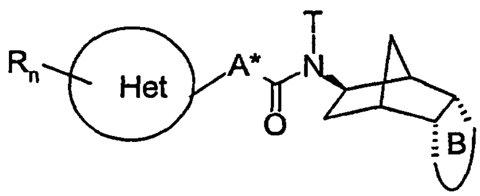

VI

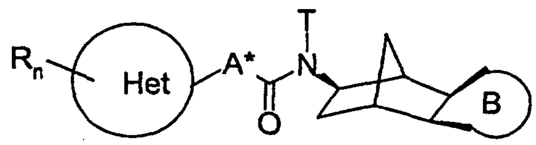

VI a

worin A* einer Bindung oder ($C_1$-$C_3$)-Alkyl entspricht und die anderen Reste, wie oben beschrieben, definiert sind,
zu den entsprechenden Aminen reduziert;
oder
e) Verbindungen der Formel I oder I a, in denen T Wasserstoff entspricht, mit Alkylierungsmitteln der Formel VII,

T*-U VII

worin T* ($C_1$-$C_4$)- Alkyl bedeutet und U oben beschriebene Bedeutung besitzt, alkyliert, so dass aus dieser Umsetzung tertiäre Amine hervorgehen;
oder
f) einen Dicyclopentadienylplatin-Komplex der Formel VIII

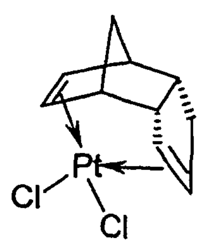

VIII

mit Aminen vom Typ der Formel IX,

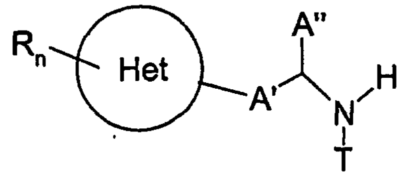

IX

worin T, $R_n$ und Het die oben angegebene Bedeutung besitzen, während unabhängig voneinander A' einer Bindung oder ($C_1$-$C_3$)-Alkyl und A" H oder ($C_1$-$C_3$)-Alkyl entspricht und A' und A" zusammen mit dem Kohlenstoffatom, an welches das Stickstoffatom gebunden ist, so viele Kohlenstoffatome repräsentieren wie das oben beschriebene A,

umsetzt und anschließend das gebildete Zwischenprodukt zu Verbindungen der Formel I reduziert und die man gegebenenfalls in das pharmazeutisch verträgliche Salz oder Trifluoracetat überführt.

**7.** Verwendung einer Verbindung I oder I a nach Anspruch 1 zur Herstellung eines Medikaments zur Behandlung oder Prophylaxe von Störungen des Atemantriebs.

**8.** Verwendung einer Verbindung I oder I a nach Anspruch 1 zur Herstellung eines Medikaments zur Behandlung oder Prophylaxe von Atemstörungen, insbesondere Schlaf-bedingten Atemstörungen wie Schlafapnoen.

**9.** Verwendung einer Verbindung I oder I a nach Anspruch 1 zur Herstellung eines Medikaments zur Behandlung oder Prophylaxe des Schnarchens.

**10.** Verwendung einer Verbindung I oder I a nach Anspruch 1 zur Herstellung eines Medikaments zur Behandlung oder zur Prophylaxe von akuten und chronischen Nierenerkrankungen, besonders des akuten Nierenversagens und des chronischen Nierenversagens.

**11.** Verwendung einer Verbindung I oder I a nach Anspruch 1 zur Herstellung eines Medikaments zur Behandlung oder Prophylaxe von Störungen der Darmfunktion.

**12.** Verwendung einer Verbindung 1 oder I a nach Anspruch 1 zur Herstellung eines Medikaments zur Behandlung oder Prophylaxe von Störungen der Gallenfunktion.

**13.** Verwendung einer Verbindung I oder I a nach Anspruch 1 zur Herstellung eines Medikaments zur Behandlung oder Prophylaxe von ischämischen Zuständen des peripheren und zentralen Nervensystems und des Schlaganfalls.

**14.** Verwendung einer Verbindung I oder I a nach Anspruch 1 zur Herstellung eines Medikaments zur Behandlung oder Prophylaxe von ischämischen Zuständen peripherer Organe und Gliedmaßen.

**15.** Verwendung einer Verbindung I oder I a nach Anspruch 1 zur Herstellung eines Medikaments zur Behandlung von Schockzuständen.

**16.** Verwendung einer Verbindung I oder I a nach Anspruch 1 zur Herstellung eines Medikaments zu Einsatz bei chirurgischen Operationen und Organtransplantationen.

**17.** Verwendung einer Verbindung I oder I a nach Anspruch 1 zur Herstellung eines Medikaments zur Konservierung und Lagerung von Transplantaten für chirurgische Maßnahmen.

**18.** Verwendung einer Verbindung I oder I a nach Anspruch 1 zur Herstellung eines Medikaments zur Behandlung von Krankheiten, bei denen die Zellproliferation eine primäre oder sekundäre Ursache darstellt.

**19.** Verwendung einer Verbindung I oder I a nach Anspruch 1 zur Herstellung eines Medikaments zur Behandlung oder Prophylaxe von Störungen des Fettstoffwechsels.

**20.** Verwendung einer Verbindung I oder I a nach Anspruch 1 zur Herstellung eines Medikaments zur Behandlung oder Prophylaxe des Befalls durch Ektoparasiten.

**21.** Heilmittel, enthaltend eine wirksame Menge einer Verbindung I oder I a nach einem oder mehreren der Ansprüche 1 bis 5.

## Claims

**1.** A substituted heterocyclo-norbomylamino derivative having an exo-configuration nitrogen and endo-fused five-membered or six-membered ring of the formula I or having an exo-configuration nitrogen and exo-fused five-membered or six-membered ring of the formula I a,

I

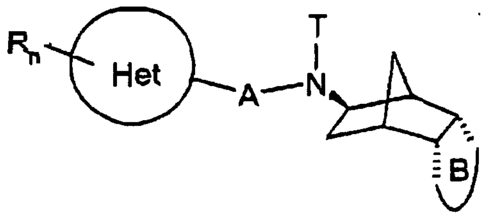

I a

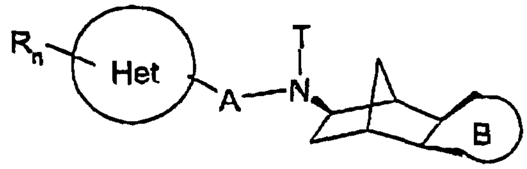

in which:

A is ($C_1$-$C_4$)- alkylene;
T is ($C_1$-$C_4$)- alkyl or H;
B is a saturated or unsaturated carbon five-membered or six-membered ring, which is unsubstituted or is substituted by 1 - 3 substituents selected from the group consisting of oxo, hydroxyl, ($C_1$-$C_4$)- alkoxy and ($C_1$-$C_4$)- alkyl;
Het is a 5- or 6-membered, saturated or unsaturated heterocycle, which contains up to four identical or different heteroatoms selected from the group consisting of O, S, N and Se;
R is OH, F, Cl, Br, I, CN, $NO_2$, phenyl, $CO_2$R1, ($C_1$-$C_4$)- alkyl, ($C_1$-$C_4$)- alkoxy, amino, ($C_1$-$C_4$)- alkylamino, di ($C_1$-$C_4$)- alkylamino, amino-($C_1$-$C_4$)- alkyl,
where the alkyl radicals are unsubstituted or are completely or partly substituted by fluorine;

R1 is H or ($C_1$-$C_4$)-alkyl, which is unsubstituted or completely or partly substituted by fluorine;

n is 0, 1, 2, 3 or 4,
where, if n = 2, 3 or 4, the substituents R are independent of one another;

or its pharmaceutically tolerable salts or trifluoracetates.

**2.** A compound having an exo-configuration nitrogen and endo-fused carbon five-membered or six-membered ring of the formula I or having an exo-configuration nitrogen and exo-fused carbon five-membered or six-membered ring of the formula I a as claimed in claim 1, wherein:

A is ($C_1$-$C_2$)- alkylene;
T is H or methyl;
B is a saturated or unsaturated carbon five-membered or six-membered ring;
Het is a 5- or 6-membered, saturated or unsaturated heterocycle, which contains up to three identical or different heteroatoms selected from the group consisting of O, S and N;
R is F, Cl, Br, iodine, amino, hydroxymethyl, OH, phenyl, $CO_2$R1, ($C_1$-$C_4$)- alkyl or ($C_1$-$C_4$)- alkoxy,
where the alkyl radicals are unsubstituted or completely or partly substituted by fluorine;

R1 is H or ($C_1$-$C_4$)-alkyl, where the alkyl radical is unsubstituted or completely or partly substituted by fluorine;

n is 0, 1, 2 or 3,
where, if n = 2 or 3, the corresponding substituents R are independent of one another,

or its pharmaceutically tolerable salts or trifluoracetates.

**3.** A compound having an exo-configuration nitrogen and endo-fused carbon five-membered or six-membered ring of the formula I or having an exo-configuration nitrogen and exo-fused carbon five-membered or six-membered ring of the formula I a as claimed in claims 1 or 2, wherein:

A is ($C_1$-$C_2$)- alkylene;
T is hydrogen;
B is a saturated or unsaturated carbon five-membered or six-membered ring;
Het is a 5- or 6-membered, saturated or unsaturated heterocycle, which contains up to two identical or different heteroatoms selected from the group consisting of O, S and N ;
R is F, Cl, Br, ($C_1$-$C_4$)- alkoxy or ($C_1$-$C_4$)- alkyl,
where the alkyl radicals are unsubstituted or completely or partly substituted by fluorine;
n is 0, 1 or 2, where, if n = 2, the corresponding substituents R are independent of one another,

or its pharmaceutically tolerable salts or trifluoracetates,

**4.** , A compound having an exo-configuration nitrogen and endo-fused carbon five-membered or six-membered ring of the formula I or having an exo-configuration nitrogen and exo-fused carbon five-membered ring of the formula I a as claimed in claim 1, which is:

exo/exo-(octahydro-4,7-methanoinden-5-yl)pyridin-3-ylmethylamine,
(rac)-exo/endo-(octahydro-4,7-methanoinden-5-yl)pyridin-3-ylmethylamine,
(+)-exo/endo-(octahydro-4,7-methanoinden-5-yl)pyridin-3-ylmethylamine,
(-)-exo/endo-(octahydro-4,7-methanoinden-5-yl)pyridin-3-ylmethylamine,
(rac)-exo/endo-(octahydro-4,7-methanoinden-5-yl)pyrazin-2-ylmethylamine,
(+)-exo/endo-(octahydro-4,7-methanoinden-5-yl)pyrazin-2-ylmethylamine,
(-)-exo/endo-(octahydro-4,7-methanoinden-5-yl)pyrazin-2-ylmethylamine,
exo/endo-(decahydro-1,4-methanonaphthalen-2-yl)pyrazin-2-ylmethylamine,
exo/endo-(octahydro-4,7-methanoinden-5-yl)thiophen-2-ylmethylamine,
exo/endo-(octahydro-4,7-methanoinden-5-yl)thiophen-3-ylmethylamine,
exo/endo-(3a,4,5,6,7,7a-hexahydro-3H-4,7-methanoinden-5-yl)pyridin-3-ylmethylamine,
exo/endo-(3a,4,5,6,7,7a-hexahydro-1H-4,7-methanoinden-5-yl)pyridin-3-ylmethylamine,
exo/endo-furan-3-ylmethyl-(octahydro-4,7-methanoinden-5-yl)amine,
exo/endo-furan-2-ylmethyl-(octahydro-4,7-methanoinden-5-yl)amine,
exo/endo-(decahydro-1,4-methanonaphthalen-2-yl)pyridin-3-ylmethylamine,
exo/endo-(octahydro-4,7-methanoinden-5-yl)-(1H-pyrrol-2-ylmethyl)amine,
exo/endo-(octahydro-4,7-methanoinden-5-yl)-pyrimidin-5-ylmethylamine

or their pharmaceutically tolerable salts or trifluoracetates.

**5.** A compound having an exo-configuration nitrogen and endo-fused carbon five-membered or six-membered ring of the formula I or having an exo-configuration nitrogen and exo-fused carbon five-membered ring of the formula I a as claimed in claim 1, which is:

exo/exo-(octahydro-4,7-methanoinden-5-yl)pyridin-3-ylmethylamine,
(rac)-exo/endo-(octahydro-4,7-methanoinden-5-yl)pyridin-3-ylmothylamine,
(+)-exo/endo-(octahydro-4,7-methanoinden-5-yl)pyridin-3-ylmethylamine,
(-)-exo/endo-(octahydro-4,7-methanoinden-5-yl)pyridin-3-ylmethylamine,
(rac)-exo/endo-(octahydro-4,7-methanoinden-5-yl)pyrazin-2-ylmethylamine,
(+)-exo/endo-(octahydro-4,7-methanoinden-5-yl)pyrazin-2-ylmethylamine,
exo/endo-(octahydro-4,7-methanoinden-5-yl)thiophen-2-ylmethylamine,
exo/endo-(3a,4,5,6,7,7a-hexahydro-3H-4,7-methanoinden-5-yl)pyridin-3-ylmethylamine,
exo/endo-(3a,4,5,6,7,7a-hexahydro-1H-4,7-methanoinden-5-yl)pyridin-3-ylmethylamine,
exo/enda-(decahydro-1,4-methanonaphthalen-2-yl)pyridin-3-ylmethylamine,

exo/endo-(octahydro-4,7-methanoinden-5-yl)-(1H-pyrrol-2-ylmethyl)amine,
exo/endo-(octahydro-4,7-methanoinden-5-yl)-pyrimidin-5-ylmethylamine

or their pharmaceutically tolerable salts or trifluoracetates,

**6.** A process for the preparation of the compounds of the formula I or I a as claimed in claim 1, which comprises

a) reacting a compound of the formula II or II a

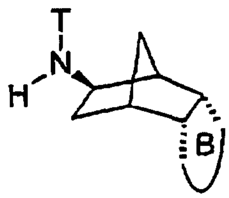
II

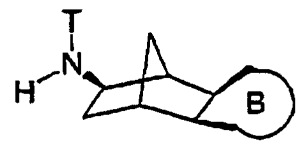
II a

with a compound of the formula III

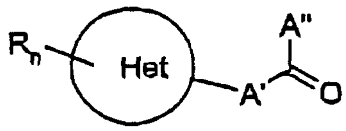
III

in the presence of suitable reductants and possibly also Lewis acids directly to give compounds of the formula I or I a,
in which T, B, Het and $R_n$ have the meaning indicated above, while independently of one another A' corresponds to a bond or ($C_1$-$C_3$)- alkyl and A" to H or ($C_1$-$C_3$)- alkyl and A' and A" together with the carbon atom of the carbonyl group represent as many carbon atoms as A described above;
or
b) isolating the intermediate, formed from compounds of the formulae II or II a and III, of the formula IV or IV a,
c)

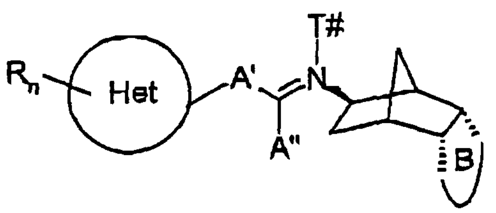
IV

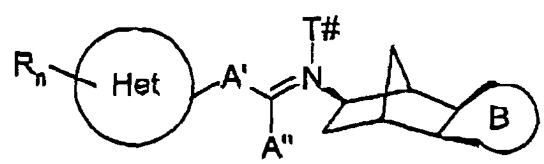
IV a

in which T# is a free electron pair or ($C_1$-$C_4$)-alkyl and in the case of the latter meaning an onium nitrogen is formed, to which is assigned a counterion, such as chloride or tosylate,
and then converting using suitable reductants into the compounds of the formula I or I a,
or
c) reacting a compound of the formula II or II a with an alkylating agent of the formula V,

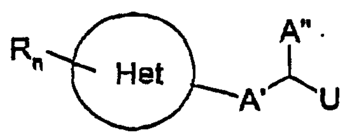
V

in which U is a nucleophilically substitutable group - such as, for example. halogen, atkyl- or arylsulfonates - and the other radicals are defined as described above, but here the carbon atom to which U is bonded corresponds to the carbon atom of the carbonyl group,

or

d) reducing carboxamides of the formula VI or VI a

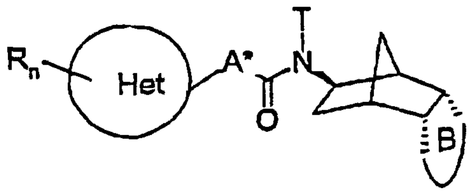

VI

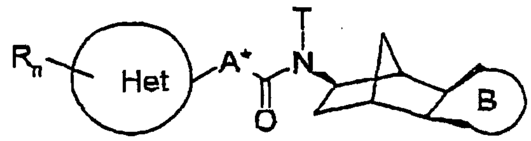

VI a

in which A* corresponds to a bond or ($C_1$-$C_3$)-alkyl and the other radicals are defined as described above, to the corresponding amines;

or

e) alkylating compounds of the formula I or I a, in which T corresponds to hydrogen, using alkylating agents of the formula VII,

T*-U VII

in which T* is ($C_1$-$C_4$)- alkyl and U has the meaning described above, such that tertiary amines result from this reaction;

or

f) reacting a dicyclopentadienylplatinum complex of the formula VIII

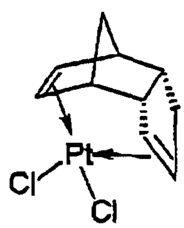

VIII

with amines of the formula IX,

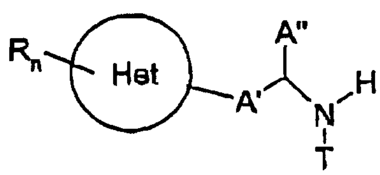

IX

in which T, $R_n$ and Het have the meaning indicated above, while independently of one another A' corresponds to a bond or ($C_1$-$C_3$)-alkyl and A" to H or ($C_1$-$C_3$)-alkyl and A' and A", together with the carbon atom to which the nitrogen atom is bonded, represent as many carbon atoms as A described above,

and subsequently reducing the intermediate formed to compounds of the formula I which are optionally converted into the pharmaceutically tolerable salt or trifluoracetate.

**7.** The use of a compound I or I a as claimed in claim 1 for the production of a medicament for the treatment or

prophylaxis of disorders of the respiratory drive.

**8.** The use of a compound I or I a as claimed in claim 1 for the production of a medicament for the treatment or prophylaxis of respiratory disorders, in particular sleep-related respiratory disorders such as sleep apnea.

**9.** The use of a compound I or I a as claimed in claim 1 for the production of a medicament for the treatment or prophylaxis of snoring.

**10.** The use of a compound I or I a as claimed in claim 1 for the production of a medicament for the treatment or for the prophylaxis of acute and chronic kidney disorders, particularly of akute kidney failure and of chronic kidney failure.

**11.** The use of a compound I or I a as claimed in claim 1 for the production of a medicament for the treatment or prophylaxis of disorders of the intestinal function.

**12.** The use of a compound I or I a as claimed in claim 1 for the production of a medicament for the treatment or prophylaxis of disorders of the bile function.

**13.** The use of a compound I or I a as claimed in claim 1 for the production of a medicament for the treatment or prophylaxis of ischemic conditions of the peripheral and central nervous system and of a stroke.

**14.** The use of a compound I or I a as claimed in claim 1 for the production of a medicament for the treatment or prophylaxis of ischemic conditions of peripheral organs and limbs.

**15.** The use of a compound I or I a as claimed in claim 1 for the production of a medicament for the treatment of states of shock.

**16.** The use of a compound I or I a as claimed in claim 1 for the production of a medicament for use in surgical operations and organ transplantation.

**17.** The use of a compound I or I a as claimed in claim 1 for the production of a medicament for the conservation and storage of transplants for surgical measures.

**18.** The use of a compound I or I a as claimed in claim 1 for the production of a medicament for the treatment of diseases in which cell proliferation is a primary or secondary cause.

**19.** The use of a compound I or I a as claimed in claim 1 for the production of a medicament for the treatment or prophylaxis of disorders of lipid metabolism.

**20.** The use of a compound I or I a as claimed in claim 1 for the preparation of a medicament for the treatment or prophylaxis of attack by ectoparasites.

**21.** A therapeutic, comprising an efficacious amount of a compound I or I a as claimed in one or more of claims 1 to 5.

**Revendications**

**1.** Dérivés hétérocyclo-norbonylamino à atome d'azote en configuration exo et cycle pentagonal ou hexagonal enda-condensé de formule I ou à atome d'azote en configuration exo et cycle pentagonal ou hexagonal exo-condensé de formule Ia

I.

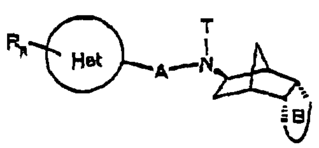

I a

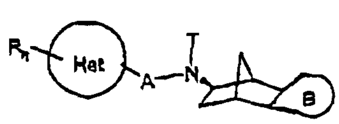

formules dans lesquelles :

A représente un groupe alkylène en $C_1$-$C_4$ ;
T représente un groupe alkyle en $C_1$-$C_4$ ou H ;
B représente un cycle carbocyclique pentagonal ou hexagonal saturé ou insaturé qui est non substitué ou porte de 1 à 3 substituants choisis dans l'ensemble consistant en des groupes oxo, hydroxy, alcoxy en $C_1$-$C_4$ et alkyle en $C_1$-$C_4$ ;
Het représente un hétérocycle à 5 ou 6 chaînons, saturé ou insaturé, qui contient jusqu'à quatre hétéroatomes identiques ou différents, choisis dans l'ensemble constitué par O, S, N et Se ;
R représente OH, F, Cl, Br, I, CN, $NO_2$, un radical phényle, $CO_2R1$, alkyle en $C_1$-$C_4$, alcoxy en $C_1$-$C_4$, amino, alkyl ($C_1$-$C_4$) amino, dialkyl($C_1$-$C_4$)amino, aminoalkyle en $C_1$-$C_4$,
les radicaux alkyle étant non substitués ou partiellement ou totalement substitués par le fluor ;
R1 représentant H ou un groupe alkyle en $C_1$-$C_4$ qui est non substitué ou partiellement ou totalement substitué par le fluor ;
n est 0, 1, 2, 3 ou 4,
lorsque n = 2, 3 ou 4, les substituants R étant indépendants les uns des autres ;

ainsi que leurs trifluorcacétates ou sels pharmaceutiquement acceptables.

**2.** Composés à atome d'azote en configuration exo et cycle carboné pentagonal ou hexagonal endo-condensé de formule I ou à atome d'azote en configuration exo et cycle carboné pentagonal ou hexagonal exo-condensé de formule Ia selon la revendication 1, **caractérisés en ce que** dans ces formules :

A représente un groupe alkylène en $C_1$-$C_2$ ;
T représente H ou le groupe méthyle ;
B représente un cycle carbocyclique pentagonal ou hexagonal saturé ou insaturé ;
Het représente un hétérocycle à 5 ou 6 chaînons, saturé ou insaturé, qui contient jusqu'à trois hétéroatomes identiques ou différents, choisis dans l'ensemble constitué par 0, S et N ;
R représente un atome de F, Cl, Br ou iode, ou un radical amino, hydroxyméthyle, OH, phényle, $CO_2R1$, alkyle en $C_1$-$C_4$ ou alcoxy en $C_1$-$C_4$,
les radicaux alkyle étant non substitués ou partiellement ou totalement substitués par le fluor ;
R1 représentant H ou un groupe alkyle en $C_1$-$C_4$ qui est non substitué ou partiellement ou totalement substitué par le fluor ;
n est 0, 1, 2 ou 3,
lorsque n = 2 ou 3, les substituants R correspondants étant indépendants les uns des autres ;

ainsi que leurs trifluoroacétates ou sels pharmaceutiquement acceptables.

**3.** Composés à atome d'azote en configuration exo et cycle carboné pentagonal ou hexagonal endo-condensé de formule I ou à atome d'azote en configuration exo et cycle carboné pentagonal ou hexagonal exo-condensé de formule Ia selon la revendication 1 ou 2, **caractérisés en ce que** dans ces formules :

A représente un groupe alkylène en $C_1$-$C_2$ ;
T représente un atome d'hydrogène ;
B représente un cycle earbacyclique pentagonal ou hexagonal saturé ou insaturé ;
Het représente un hétérocycle à 5 ou 6 chaînons, saturé ou insaturé, qui contient jusqu'à deux hétéroatomes identiques ou différents, choisis dans l'ensemble constitué par O, S et N ;
R représente F, Cl, Br ou un radical alcoxy en $C_1$-$C_4$ ou alkyle en $C_1$-$C_4$,
les radicaux alkyle étant non substitués ou partiellement ou totalement substitués par le fluor ;
n est 0, 1 ou 2,
lorsque n = 2, les substituants R correspondants étant indépendants l'un de l'autre ;

ainsi que leurs triflucroacétates ou sels pharmaceutiquement acceptables.

**4.** Composés à atome d'azote en configuration exo et cycle carboné pentagonal ou hexagonal endo-condensé de formule I ou à atome d'azote en configuration exo et cycle carboné pentagonal ou hexagonal exo-condensé de formule Ia selon la revendication 1, **caractérisés en ce qu'**il s'agit des composés suivants :

exo/exo-(octahydro-4,7-méthano-indén-5-yl)-pyridin-3-ylméthyl-amine,
(rac)-exo/endo-(octahydro-4,7-méthano-indén-5-yl)-pyridin-3-ylntéthyl-amine,
(+)-exo/endo-(octahydro-4,7-méthano-indén-5-yl)-pyridin-3-ylméthyl-amine,
(-)-exo/endo-(octahydro-4,7-méthano-indén-5-yl)-pyridin-3-ylméthyl-amine,
(rac)-exo/endo-(octahydro-4,7-méthano-indén-5-yl)-pyrazin-2-ylméthyl-amine,
(+)-exo/endo-(octahydro-4,7-méthano-indén-5-yl)-pyrazin-2-ylméthyl-amine,
(-)-exo/endo-(octahydro-4,7-méthano-indén-5-yl)-pyrazin-2-ylméthyl-amine,
exo/endo-(décahydro-1,4-méthano-naphtalén-2-yl)-pyrazin-2-ylméthyl-amine,
exo/endo-(octahydro-4,7-méthano-indén-5-yl)-thiophén-2-ylméthyl-amine,
exo/endo-(octahydro-4,7-méthano-indén-5-yl)-thiophén-3-ylméthyl-amine,
exo/endo-(3a,4,5,6,7,7a-hexahydro-3H-4,7-méthano-indén-5-yl)-pyridin-3-yl-méthyl-amine,
exo/endo-(3a,4,5,6,7,7a-hexahydro-1H-4,7-méthano-indén-5-yl)-pyridin-3-yl-méthyl-amine,
exo/endo-furann-3-ylméthyl-(octahydro-4,7-méthano-indén-5-yl)-amine,
exo/endo-furann-2-ylméthyl-(octahydro-4,7-méthano-indén-5-yl)-amine,
exo/endo-(décahydro-1,4-méthano-naphtalén-2-yl)-pyridin-3-ylméthyl-amine,
exo/endo-(octahydro-4,7-méthano-indén-5-yl)-(1H-pyrrol-2-ylméthyl)-amine,
exo/endo-(octahydro-4,7-méthano-indén-5-yl)-pyrimidin-5-ylméthyl-amine

ainsi que leurs trifluoroacétates ou sels pharmaceutiquement acceptables.

**5.** Composés à atome d'azote en configuration exo et cycle carboné pentagonal ou hexagonal endo-condensé de formule I ou à atome d'azote en configuration exo et cycle carboné pentagonal ou hexagonal exo-condensé de formule Ia selon la revendication 1, **caractérisés en ce qu'**il s'agit des composés suivants :

exo/exo-(octahydro-4,7-méthano-indén-5-yl)-pyridin-3-ylméthyl-amine,
(rac)-exo/endo-(octahydro-4,7-méthano-indén-5-yl)-pyridin-3-ylméthyl-amine,
(+)-exo/endo-(octahydro-4,7-méthano-indén-5-yl)-pyridin-3-ylméthyl-amine,
(-)-exo/endo-(octahydro-4,7-méthano-indén-5-yl)-pyridin-3-ylméthyl-amine,
(rac)-exo/endo-(octahydro-4,7-méthano-indén-5-yl)-pyrazin-2-ylméthyl-amine,
(+)-exo/endo-(octahydro-4,7-méthano-indén-5-yl)-pyrazin-2-ylméthyl-amine,
exo/endo-(octahydro-4,7-méthano-indén-5-yl)-thiophén-2-ylméthyl-amine,
exo/endo-(3a,4,5,6,7,7a-hexahydro-3H-4,7-méthano-indén-5-yl)-pyridin-3-yl-méthyl-amine,
exo/endo-(3a,4,5,6,7,7a-hexahydro-1H-4,7-méthano-indén-5-yl)-pyridin-3-yl-méthylamine,
exo/endo-(décahydro-1,4-méthano-naphtalén-2-yl)-pyridin-3-ylméthyl-amine,
exo/endo-(octahydro-4,7-méthano-indén-5-yl)-(1H-pyrrol-2-ylméthyl)-amine,
exo/endo-(octahydro-4,7-méthano-indén-5-yl)-pyrimidin-5-ylméthyl-amine

ainsi que leurs trifluoroacétates ou sels pharmaceutiquement acceptables.

**6.** Procédé pour la préparation des composes de formule I ou Ia selon la revendication 1, **caractérisé en ce que**

a) on fait réagir un composé de formule II ou IIa

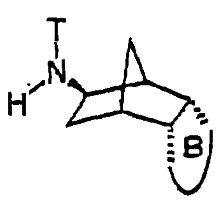

II .

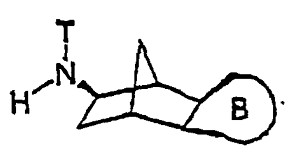

II a

avec un composé de formula III

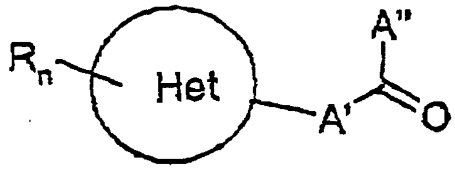

III

en présence de réducteurs appropriés et éventuellement aussi d'acides de Lewis, pour aboutir directement aux composés de formule I ou Ia,

dans lesquels T, B, Het et $R_n$ ont les significations données plus haut, tandis que, indépendamment l'un de l'autre, A' correspond à une liaison ou à un groupe alkyle en $C_1$-$C_3$ et A" correspond à H ou à un groupe alkyle en $C_1$-$C_3$ et A' et A" représentent ensemble, avec l'atome de carbone du groupe carbonyle autant d'atomes de carbone que le groupe A décrit plus haut ;

ou

b) on isole le composé intermédiaire de formule IV ou IVa,

c)

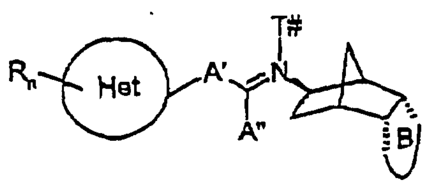

IV

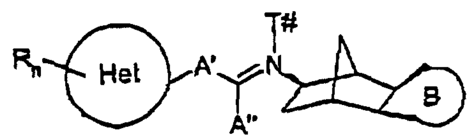

IV a

formules dans lesquelles T# correspond à une paire d'électrons libres ou à un groupe alkyle en $C_1$-$C_4$ et, dans le cas de cette dernière signification, il se forme un atome d'azote en fonction onium auquel est affecté un ion opposé, tel que chlorure ou toaylate,

formé à partir de composés de formule II ou IIa et III,

et on le convertit ensuite, à l'aide de réducteurs appropriés, en les composés de formule I ou Ia,

ou

c) on fait réagir un composé de formule II ou IIa avec un agent d'alkylation de formule V,

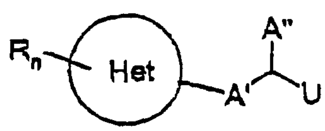

V

dans laquelle U représente un groupe pouvant être remplacé par substitution nucléophile - comme par exemple halogéno, alkyl- ou arylsulfonates - et les autres radicaux sont définis comme décrit plus haut, mais en ce cas l'atome de carbone du groupe carbonyle correspond à l'atome de carbone auquel U est lié,

ou

d) on réduit en les amines correspondantes des carboxamides de formule VI ou VIa

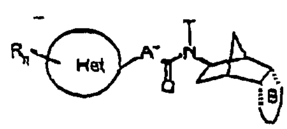

VI

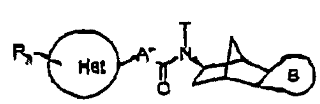

VI a

formules dans lesquelles A* correspond à une liaison ou à un groupe alkyle en $C_1$-$C_3$ et les autres radicaux sont définis comme décrit plus haut ;
ou
e) on soumet des composés de formule I ou Ia, dans lesquelles T représente un atome d'hydrogène, à une alkylation avec des agents d'alkylation de formule VII,

T*-U VII

dans laquelle T* représente un groupe alkyle en $C_1$-$C_4$ et U a la signification décrite plus haut, de manière que des amines tertiaires résultent de cette réaction ;
ou
f) on fait réagir un complexe de dicyclopentadiénylplatine de formule VIII

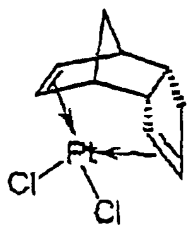

VIII

avec des amines du type de formule IX,

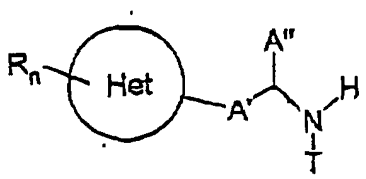

IX

dans laquelle T, $R_n$ et Het ont les significations données plus haut, tandis que, indépendamment l'un de l'autre, A' correspond à une liaison ou à un groupe alkyle en $C_1$-$C_3$ et A" correspond à H ou à un groupe alkyle en $C_1$-$C_3$ et A' et A" représentent ensemble, avec l'atome de carbone auquel est lié l'atome d'azote, autant d'atomes de carbone que le groupe A décrit plus haut,

et on réduit ensuite le produit intermédiaire formé en composés de formule I
et éventuellement on les convertit en le trifluoroacétate ou sel pharmaceutiquement acceptable.

**7.** Utilisation d'un composé I ou Ia selon la revendication 1, pour la fabrication d'un médicament destiné au traitement ou à la prophylaxie de troubles de l'impulsion respiratoire.

**8.** Utilisation d'un composé I ou Ia selon la revendication 1, pour la fabrication d'un médicament destiné au traitement ou à la prophylaxie de troubles respiratoires, en particulier de troubles respiratoires dus au sommeil, tels que les apnées du sommeil.

**9.** Utilisation d'un composé I ou Ia selon la revendication 1, pour la fabrication d'un médicament destiné au traitement ou à la prophylaxie du ronflement.

**10.** Utilisation d'un composé I ou Ia selon la revendication 1, pour la fabrication d'un médicament destiné au traitement ou à la prophylaxie de néphropathies aigués et chroniques, en particulier de l'insuffisance rénale aigué et de l'insuffisance rénale chronique.

**11.** Utilisation d'un composé I ou Ia selon la revendication 1, pour la fabrication d'un médicament destiné au traitement ou à la prophylaxie de troubles de la fonction intestinale.

**12.** Utilisation d'un composé I ou Ia selon la revendication 1, pour la fabrication d'un médicament destiné au traitement ou à la prophylaxie de la fonction biliaire.

**13.** Utilisation d'un composé I ou Ia selon la revendication 1, pour la fabrication d'un médicament destiné au traitement ou à la prophylaxie d'états ischémiques du système nerveux central et périphérique et de l'accident vasculaire cérébral.

**14.** Utilisation d'un composé I ou Ia selon la revendication 1, pour la fabrication d'un médicament destiné au traitement ou à la prophylaxie d'états ischémiques des organes périphériques et des membres.

**15.** Utilisation d'un composé I ou Ia selon la revendication 1, pour la fabrication d'un médicament destiné au traitement d'états de choc.

**16.** Utilisation d'un composé I ou Ia selon la revendication 1, pour la fabrication d'un médicament destiné à l'utilisation dans des opérations chirurgicales et des transplantations d'organes.

**17.** Utilisation d'un composé I ou Ia selon la revendication 1, pour la fabrication d'un médicament destiné à la conservation et au stockage de transplants pour des interventions chirurgicales.

**18.** Utilisation d'un composé I ou Ia selon la revendication 1, pour la fabrication d'un médicament destiné au traitement ou à la prophylaxie de maladies dans lesquelles la prolifération cellulaire représente une cause primaire ou secondaire.

**19.** Utilisation d'un composé I ou Ia selon la revendication l, pour la fabrication d'un médicament destiné au traitement ou à la prophylaxie de troubles du métabolisme lipidique.

**20.** Utilisation d'un composé I ou Ia selon la revendication 1, pour la fabrication d'un médicament destiné au traitement ou à la prophylaxie de l'attaque par des ectoparasites.

**21.** Médicament, contenant une quantité efficace d'un composé I selon une ou plusieurs des revendications 1 à 5.